# FASCICULE DE BREVET EUROPEEN

(11) **EP 2 780 333 B1**
(45) Date de publication et mention de la délivrance du brevet: **30.05.2018**
(21) Numéro de dépôt: 12787448.5
(22) Date de dépôt: 16.11.2012
(51) Int. Cl.: C07D 257/02, C07D 487/04, C07D 487/08, C07D 487/22

(54) **PROCÉDÉS DE PRÉPARATION DE COMPOSÉS TÉTRAAZACYCLOALCANES FONCTIONNELS À L'AIDE D'UN COMPOSÉ CYCLIQUE BISAMINAL PARTICULIER**
VERFAHREN ZUR HERSTELLUNG FUNKTIONELLER TETRAAZACYCLOALKAN-VERBINDUNGEN MITTELS EINER SPEZIFISCHEN CYCLISCHEN BISAMINALVERBINDUNG
METHODS FOR PREPARING FUNCTIONAL TETRAAZACYCLOALKANE COMPOUNDS USING A SPECIFIC CYCLIC BISAMINAL COMPOUND

(30) Priorité: 16.11.2011 FR 1160426
(43) Date de publication de la demande: 24.09.2014
(73) Titulaire: Université de Bretagne Occidentale, 29238 Brest Cedex 3 (FR); Centre National de la Recherche Scientifique CNRS, 75794 Paris Cedex 16 (FR)
(72) Inventeur: TRIPIER, Raphaël, F-29860 Kersaint Plabennec (FR); CAMUS, Nathalie, F-29200 Brest (FR)
(74) Mandataire: Vidon Brevets & Stratégie
(86) Numéro de dépôt international: PCT/EP2012/072898
(87) Numéro de publication internationale: WO 2013/072491

(56) Documents cités:
- US-A1- 2006 217 548
- US-B2- 7 312 327
- MARINE ANTOINE ET AL: "Synthesis and NMR characterisation of new cyclam-glyoxal diamides", JOURNAL OF THE CHEMICAL SOCIETY, PERKIN TRANSACTIONS 2, no. 3, 25 février 2002 (2002-02-25), pages 552-555, XP055023975, ISSN: 1472-779X, DOI: 10.1039/b109307j
- TAKENOUCHI K ET AL: "Novel Bifunctional Macrocyclic Chelating agents Appended with a Pendant-Type Carboxymethylamino Ligand and Nitrobenzyl Group and Stability of the 88Y(III) Complexes", JOURNAL OF ORGANIC CHEMISTRY, AMERICAN CHEMICAL SOCIETY, EASTON.; US, vol. 58, no. 7, 1 janvier 1993 (1993-01-01), pages 1955-1958, XP002399531, ISSN: 0022-3263, DOI: 10.1021/JO00059A062

## Description

Le domaine de l'invention est celui de la synthèse de composés tétraazacycloalcanes fonctionnels, en particulier de dérivés du cyclam. Ces composés peuvent être polyfonctionnels et notamment bi-fonctionnels.

Plus précisément, l'invention concerne un composé cyclique bisaminal particulier à partir duquel dérivent des procédés de préparation de dérivés du cyclam selon l'invention, ainsi que les procédés de préparation de ce composé.

La chimie des polyamines cycliques et en particulier des tétraazacycloalcanes dont les deux représentants principaux sont le 1,4,7,10-tétraazacyclododécane (cyclène) et le 1,4,8,11-tétraazacyclotétradécane (cyclam) a connu un développement considérable au cours de ces dernières décennies.

Grâce aux doublets libres de ses amines secondaires, ces macrocycles azotés peuvent former des complexes très stables avec de nombreux cations et notamment les métaux de transition. La capacité chélatante de ces composés peut être améliorée, ou encore être étendue à d'autres métaux tels que les lanthanides ou les métaux lourds, à l'aide de groupes fonctionnels et en particulier de ligands particuliers, présentant une affinité avec ces cations ou métaux. En fonctionnalisant ces macrocycles, c'est-à-dire en y greffant un ou plusieurs groupes fonctionnels notamment coordinants (ligands), il est possible de modifier les propriétés complexantes du macrocycle, son affinité et sa sélectivité par rapport à un élément donné, sa solubilité. Il est ainsi possible d'obtenir par exemple des molécules capables d'être greffées sur un support solide, sur une nanoparticule, ou d'être vectorisées vers des cellules ou organes cibles, par exemple par greffage sur un vecteur biologique tel qu'un anticorps, un haptène ou un peptide. Il est également possible de conférer à ces macrocycles des propriétés de luminescence telle que la fluorescence.

Selon les propriétés recherchées, les dérivés du cyclam et du cyclène obtenus peuvent être utilisés dans de nombreuses applications, dans des domaines très variés, tels que la catalyse, le magnétisme, les matériaux, l'épuration de liquides et de gaz, les méthodes d'analyse et de détection, la médecine, notamment la médecine nucléaire.

Les dérivés du cyclam et du cyclène dit « cross bridge » et « side-bridge » qui correspondent aux tétraazacyclododécanes ayant une structure pontée (pont éthylène), respectivement entre deux atomes d'azote opposés et adjacents du macrocycle, représentent notamment une cible intéressante dans le domaine thérapeutique, en particulier dans le développement de radiopharmaceutiques. De part leur structure particulière, ils sont capables de former un complexe métallique très stable sans "relargage" du métal dans l'organisme.

Les macrocycles azotés peuvent être fonctionnalisés de trois manières différentes: soit directement sur un des atomes d'azote (N-fonctionnalisation) du macrocycle, soit sur un ligand relié à un des atomes d'azote du macrocycle, soit sur un atome de carbone (C-fonctionnalisation) du macrocycle. Toutefois, la première voie de synthèse présente l'inconvénient de conduire à la perte ou à la réduction des propriétés chélatantes du macrocycle en rendant indisponible un ou plusieurs doublets libres des atomes d'azote du macrocycle, et ne permet pas d'accéder à des dérivés « cross-bridge » et « side-bridge » fonctionnalisés. La seconde voie de synthèse présente l'inconvénient de modifier ou d'entraver la fonction du ligand issu d'une première fonctionnalisation du macrocycle. Nos travaux se sont donc orientés vers la dernière voie de synthèse qui présente l'avantage par rapport aux deux autres voies de synthèse, de pouvoir greffer un groupe fonctionnel permettant par exemple de coupler le macrocycle à un support (fonction de couplage), sans modifier les propriétés chélatantes du macrocycle ou dénaturer la structure et les propriétés d'autres ligands reliés au macrocycle. Cette voie de synthèse est particulièrement intéressante pour obtenir des macrocycles polyfonctionnels, notamment bifonctionnels, capables de chélater un atome ou cation métallique d'une part et d'être greffé sur un élément donné (support, nanoparticule, biomolécule) d'autre part, c'est-à-dire possédant au moins une fonction de coordination et au moins une fonction de couplage. Cette dernière voie de synthèse, qui paraît simple en théorie, révèle plusieurs difficultés de mise en oeuvre pratique.

Les méthodes les plus usuelles et les plus anciennes reposent sur une cyclisation directe des macrocycles à partir de synthons pré-fonctionnalisés. Ces méthodes présentent notamment l'inconvénient de ne pas être généralisables quant à la taille du macrocycle et la nature de la fonction de couplage, ce qui constitue un inconvénient pour une mise en oeuvre à l'échelle industrielle. Certaines de ces méthodes sont décrites par exemple dans la demande de brevet WO 03/029228.

En particulier, il est connu de cette demande de brevet WO 03/029228 de préparer des dérivés du cyclam à partir d'une tétraamine et d'un composé dicarbonylé. L'intermédiaire bisaminal obtenu est ensuite cyclisé à l'aide d'un réactif dihalogéné en présence de K₂CO₃ dans de l'acétonitrile. Si ce procédé permet d'obtenir un rendement de cyclisation satisfaisant (supérieur ou égal à 50%), il présente cependant l'inconvénient d'employer d'importantes quantités d'acétonitrile, rendant le procédé cher, peu respectueux de l'environnement, et peu adapté à une transposition à l'échelle industrielle.

D'autres documents de l'art antérieur décrivant des procédés de préparation de tétraazacycloalcanes C-fonctionnalisés sont notamment TAKENOUCHI K. et AL (« Novel Bifunctional Macrocyclic Chelating agents Appended with a Pendant-Type Carboxymethylamino Ligand and Nitrobenzyl Group and Stability of the 88Y(III) Complexes, JOC, vol.58, no 7, pages 1955-1958) et la demande de brevet US 7 312 327.

Le demandeur a maintenant découvert de nouveaux procédés de préparation de composés tétraazacycloalcanes fonctionnels, en particulier de dérivés du cyclam, et plus particulièrement des procédés de préparation de composés tétraazacycloalcanes fonctionnels ne présentant pas les inconvénients de l'art antérieur, qui soient notamment plus respectueux de l'environnement, intéressant économiquement (coût des solvants, coût en énergie), simples à mettre en oeuvre et qui conviennent à une mise en oeuvre à l'échelle industrielle.

La plupart des étapes des procédés selon l'invention, notamment celles pour cycliser le composé tétraazoté, peuvent être effectuées dans des solvants protiques tels que de préférence l'eau et/ou le méthanol, en faible quantité, à des températures relativement basses (inférieures ou égales à 100°C, de préférence inférieures à 60°C, et mieux encore inférieures à 50°C).

Ces procédés selon l'invention sont ainsi plus respectueux de l'environnement, ils nécessitent peu d'énergie pour être mis en oeuvre.

Les procédés selon l'invention comportent par ailleurs un nombre d'étapes limité.

Ils ne nécessitent aucune étape de purification délicate.

Ils conduisent à des rendements, notamment de cyclisation, satisfaisants (au moins 50%).

Avantageusement, la cyclisation de ces composés peut être réalisée en « one pot », c'est-à-dire *in situ* dans un même milieu réactionnel. En particulier, elle peut se faire sans séparation et/ou sans purification intermédiaire.

Un premier objet de l'invention porte donc sur un composé cyclique bisaminal C-fonctionnalisé et son procédé de préparation. L'obtention de ce composé constitue une étape clé dans la synthèse de composés tétraazacycloalcanes fonctionnels, et notamment de dérivés du cyclam, notamment bifonctionnels capable de chélater un atome ou cation m&tallique et d'être greffé sur un substrat donné. Il permet à la fois de cycliser le macrocycle et de greffer un groupe fonctionnel au niveau de son squelette carboné.

Un second objet de l'invention porte sur des procédés de préparation de composés cycliques C et/ou N-fonctionnalisés particuliers, mettant en oeuvre un composé cyclique bisaminal C-fonctionnalisé selon l'invention.

D'autres objets, aspects, caractéristiques ou avantages de l'invention apparaîtront plus clairement au vu de la description et des exemples.

Par « cyclique », on entend que le composé comprend dans sa structure au moins un groupe cyclique, ledit cycle pouvant être saturé ou insaturé, aromatique ou non aromatique, comprenant de préférence de 5 à 6 membres, tel que par exemple un groupe phényle ou pyrrole. Sauf mention contraire dans la présente demande, on utilisera ainsi le terme cyclique pour désigner indifféremment mono ou polycyclique.

Le composé cyclique bisaminal C-fonctionnalisé selon l'invention répond à la formule (I) ou (II), ses solvates ou un de ses sels: 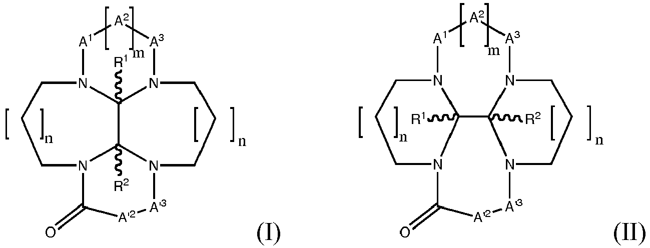 dans lesquelles :
R¹ et R², identiques ou différents, représentent un atome d'hydrogène, un groupe alkyle en C₁-C₄, de préférence méthyle, ou peuvent former ensemble avec les atomes de carbone du pont bisaminal auxquels ils sont reliés un groupe cyclique hydrocarboné à 6 membres, saturé ou insaturé, tel qu'un groupe phényle ou cyclohexane, de préférence cyclohexane,
m et n, identiques ou différents, sont égaux à zéro ou à un ;
A¹, A², A³, A'², A'³, identiques ou différents, sont choisis parmi les groupes CH₂, CHR et C=O ; sous réserve que A'² ou A'³ désigne un groupe CHR ;
le ou les groupes R, identiques ou différents, pouvant désigner n'importe quelle groupe fonctionnel, ou précurseur de groupe fonctionnel.

Par groupe fonctionnel, on entend un groupe conférant des propriétés particulières au composé cyclique, comme décrites par exemple dans le préambule de la présente demande, à savoir un groupe capable de modifier les propriétés complexantes du composé cyclique, son affinité et sa sélectivité par rapport à un élément donné, sa solubilité ; de greffer ou coupler le composé cyclique sur un support (solide, nanoparticule, vecteur biologique) ; de conférer des propriétés de luminescence. En particulier, le groupe fonctionnel peut être une fonction de couplage, c'est-à-dire une fonction réactive permettant de créer une liaison carbone-carbone ou hétéroatome-carbone stable. Le groupe fonctionnel peut posséder ou non une ou plusieurs fonctions de coordination, conférant à la molécule porteuse du groupe fonctionnel des propriétés complexantes éventuellement améliorée par rapport à un substrat donné.Le groupe fonctionnel possédant une ou plusieurs fonctions coordinantes est aussi appelé ligand.

Par précurseur de groupe fonctionnel, on entend un groupe nécessitant d'être transformé par une ou plusieurs réactions chimiques, par exemple une alkylation ou un traitement en milieu acide, pour obtenir un groupe fonctionnel.

Dans les formules (I) ou (II), de préférence m est égal à un.

De préférence, R¹ et R², sont choisis parmi un atome d'hydrogène et un groupe méthyle, ou bien forment ensemble avec les atomes de carbone du pont bisaminal auxquels ils sont reliés un groupe cyclohexane. Plus préfentiellement, ils sont choisis parmi un atome d'hydrogène et un groupe méthyle.

Selon un premier mode de réalisation, A'³ désigne un groupe CHR.

Selon un second mode de réalisation, A'² désigne un groupe CHR.

De référence, on utilise un composé de formule (III) suivante, un de ses sels ou solvates : 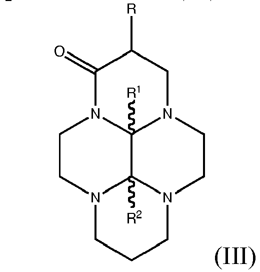 dans laquelle : R, R¹, R² sont els que définis dans les formules (I) et (II).
- Le ou les groupes R des formules (I), (II) ou (III) peuvent être ioniques ou non ioniques.

En particulier, ils peuvent être choisis, indépendamment les uns des autres, parmi :
- un groupe hydrocarboné, saturé ou insaturé, linéaire ou ramifié, cyclique ou acyclique, comprenant de 1 à 30 atomes de carbone, de préférence linéaire et de 8 à 20 atomes de carbone, et comprenant éventuellement un ou plusieurs hétéroatomes, tels que O, N, S, P ou un halogène,
- un groupe V, V étant choisi parmi un halogène, un nitrile, NR⁵R⁶, NO₂, OR⁷, COX, NHCOX, -CONR⁵R⁶, (O)ₛPO(OR⁸)(OR⁹), s étant un entier allant de zéro à un, X étant un halogène ou un groupe OR⁵; R⁵, R⁶, R⁷, R⁸ et R⁹ étant choisis parmi un atome d'hydrogène et un groupe alkyle, linéaire ou ramifié, saturé ou insaturé, en C₁-C₃₀,

- un groupe W, W étant un groupe hydrocarboné cyclique pouvant être mono, bi ou tricyclique, saturé ou insaturé, le ou les cycles comportant de 5 à 6 membres, et au moins l'un d'eux pouvant être substitué par un ou plusieurs groupes choisis parmi N-chlorosuccinimide, P(C₆H₅)₂, P(O)(C₆H₅)₂, (CH₂)ₚV, p étant un entier allant de zéro à quatre, et/ou pouvant former avec un ou plusieurs atomes de carbone du cycle une ou plusieurs fonctions cétone,
- un groupe Y, Y étant un groupe hétérocyclique pouvant être mono, bi ou tricyclique, saturé ou insaturé, le ou les cycles comportant de 5 à 6 membres, et au moins l'un d'eux pouvant être substitué sur un ou plusieurs atomes de carbone ou hétéroatome par un ou plusieurs groupes choisis parmi les groupes V et W, un groupe alkyle linéaire ou ramifié, saturé ou insaturé, de préférence en C₁-C₃₀, substitué par un ou plusieurs groupes V et/ou W, un groupe trityle ; ledit groupe hétérocycle pouvant former avec un ou plusieurs atomes de carbone du cycle une ou plusieurs fonctions cétone,
- un groupe alkyle en C₁-C₃₀, linéaire ou ramifié, saturé ou insaturé, substitué par un ou plusieurs groupes V, W, et/ou Y.

De préférence, le ou les groupes R sont choisis selon les propriétés ou fonctions que l'on souhaite conférer au composé (I), (II) ou (III). R peut être choisi de manière à augmenter l'affinité, la sélectivité du cyclène ou du cyclam avec un élément donné, tel qu'un cation, un complexe anionique ou un atome métallique, un support solide organique ou inorganique, une nanoparticule, une biomolécule (anticorps, haptène, peptide, sucre...).

A titre d'exemple, on peut choisir R parmi :
- des groupes hydrophobes, tels que par exemple des groupes hydrocarbonés, saturés ou insaturés, linéaires ou ramifiés, cycliques ou acycliques, comprenant au moins une chaîne hydrocarboné linéaire ayant de 8 à 30 atomes de carbone,
- des groupes hydrophiles, tels que ceux comportant au moins un groupe éthylène glycol, par exemple l'éthylène glycol ou polyéthylène glycol,
- des groupes tensioactifs, tels que par exemple des groupes de type lipide / phospholipide,
- des groupes fluorescents, tels que par exemple anthracène, phénanthrène, phénanthroline, mésitylbodipy,
- des groupes ioniques, tels que des groupes organiques de type guadinidium ou arsenium,
- des groupes porteurs d'au moins une fonction amine primaire, secondaire ou tertiaire éventuellement protégée par les protections usuelles (Boc, tosyle (Ts)...),
- des groupes porteurs d'au moins une fonction alcool,
- des groupes porteurs d'au moins une fonction alcène et/ou alcyne,
- des groupes porteurs d'au moins une fonction amide,
- des groupes porteurs d'au moins une fonction cyano, nitrile, CF₃,
- des groupes porteurs d'au moins une fonction acide, ester, aldéhyde.

Parmi tous les composés de formule (I), (II) ou (III) utilisables, on préfère ceux dont le ou les groupes R sont choisis parmi les groupes (CH₂)ᵢX avec i un entier égal à zéro ou un, X étant un groupe choisi parmi un atome d'halogène, un hydroxyle, CH₂OH, COOH, COOCH₃, COOC₂H₅. En particulier, on préfère utiliser les composés de formule (III) dans laquelle : R¹ et R², identiques ou différents, représentent un atome d'hydrogène, un groupe alkyle en C₁-C₄, ou peuvent former ensemble avec les atomes de carbone du pont bisaminal auxquels ils sont reliés un groupe cyclique hydrocarboné à 6 atomes de carbone, saturé ou insaturé; R est un groupe (CH₂)ᵢX avec i un entier égal à zéro ou un, X étant un groupe choisi parmi un atome d'halogène, un hydroxyle, CH₂OH, COOH, COOCH₃, COOC₂H₅.

L'invention couvre aussi les stéréoisomères des composés cycliques bisaminals C-fonctionnalisés sus-décrits, en particulier, les énantiomères et les diastéréoisomères.

Les groupes R¹ et R² sont de préférence positionnés de manière à limiter les gênes stériques pouvant apparaître entre les différents substituants du cyclam et de ses dérivés. Ils peuvent être en position cis ou trans, en position endo ou exo, par rapport au squelette carboné du cyclam ou du cyclène. De préférence, les groupes R¹ et R² sont en position cis.

Selon un mode de réalisation particulier, le composé de formule (I) ou (II), selon l'invention peut comporter au moins deux fonctions amides, de préférence au moins l'un des groupes A₁, A₂ ou A₃ désigne un groupe C=O. De préférence, A₁ ou A₃ désigne un groupe C=O. Lorsque A₁ ou A₃ désigne un groupe C=O, les fonctions di-oxo du composé de formule (I) ou (II) selon l'invention sont en position cis ou trans.

Les formules suivantes servent à illustrer des composés selon l'invention présentant de telles configurations: 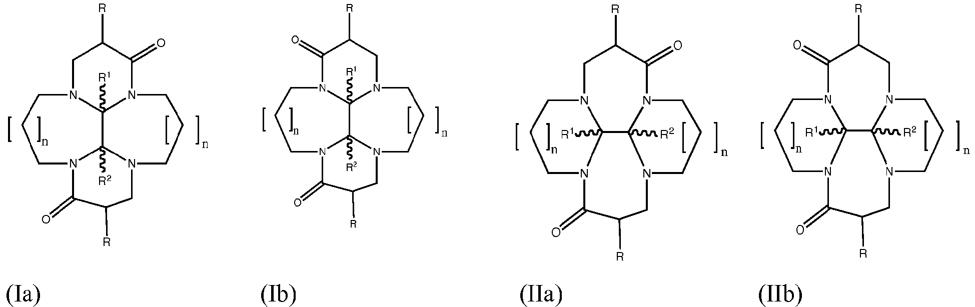 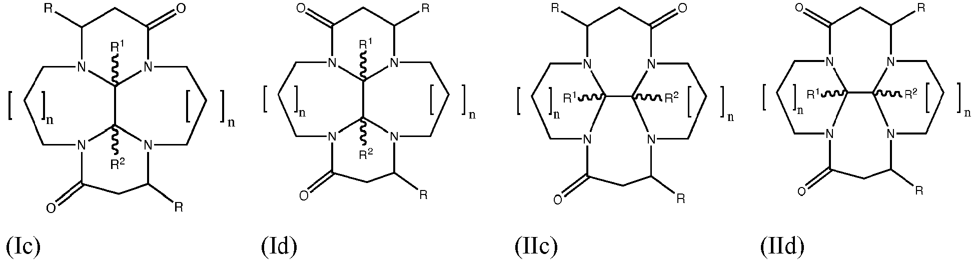 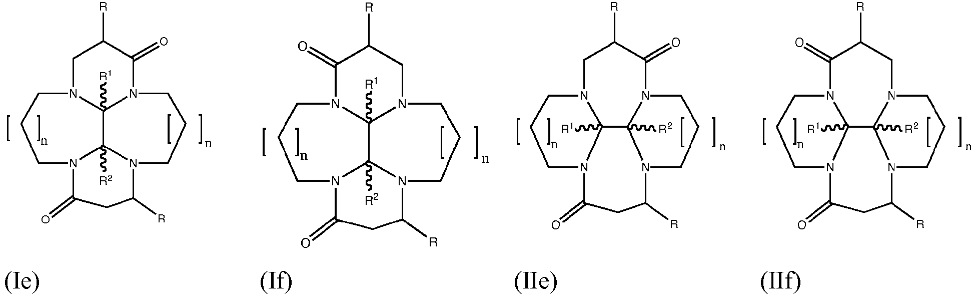 formules dans lesquelles : R, R¹ et R², n sont tels que définis dans les formules (I) ou (II).

L'invention a également pour objet un procédé de préparation d'un composé cyclique bisaminal C-fonctionnalisé tel que défini précédemment comprenant :
(i) une étape au cours de laquelle on fait réagir une tétramine de formule (VII) 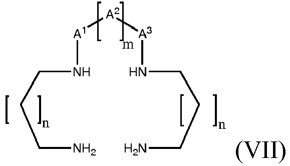
   dans laquelle : A¹, A², A³ sont choisis parmi les groupes CH₂, CHR et C=O, et de préférence des groupes CH₂,
   m et n, identiques ou différents, sont égaux à zéro ou un,
   avec un composé dicarbonyle de formule (IV) : 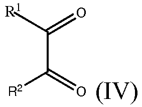 dans laquelle : R¹ et R², identiques ou différents, représentent un atome d'hydrogène, un groupe alkyle en C₁-C₄, de préférence méthyle, ou peuvent former ensemble avec les atomes de carbone auxquels ils sont reliés un groupe cyclique hydrocarboné à 6 atomes de carbone, saturé ou insaturé, tel qu'un groupe phényle ou cyclohexane, et de préférence cyclohexane,
   pour former un intermédiaire bisaminal de formule (VIII) ou (IX) ou un mélange de ces composés: 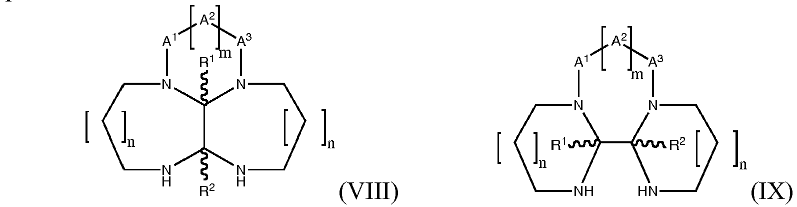 formules (VIII) et (IX) dans lesquelles : A¹, A², A³, m, n sont tels que définis dans la formule (VII) et R¹ et R² sont tels que définis dans la formule (IV) ;
(ii) puis une étape au cours de laquelle on fait réagir le ou les intermédiaires obtenus avec un agent cyclisant répondant à la formule (VI), de préférence dans un solvant protique: 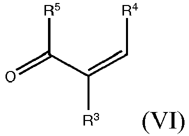 formule (VI) dans laquelle :
   R³ et R⁴, identiques ou différents, peuvent être choisis parmi un atome d'hydrogène et le groupe R tel que défini dans la formule (I) ou (II); et
   R⁵ représente un hydroxyle, un halogène, un alcoxyle tel que mésyle, tosyle ; ou
   R⁵ peut former avec R³ ou R⁴ un groupe hétérocyclique ayant de 5 à 6 membres.
   De préférence, l'étape (i) et/ou (ii) ont lieu dans un milieu (ou solvant) protique.

L'invention a aussi pour objet un procédé de préparation d'un composé cyclique bisaminal tel que défini précédemment comprenant :
(i) une étape au cours de laquelle on fait réagir au moins deux équivalents d'éthylène diamine ou de propylène diamine avec au moins un équivalent d'un composé dicarbonyle de formule (IV) tel que défini précédemment,
   pour former un intermédiaire bisaminal de formule (V) ou (V') ou un mélange de ces composés: 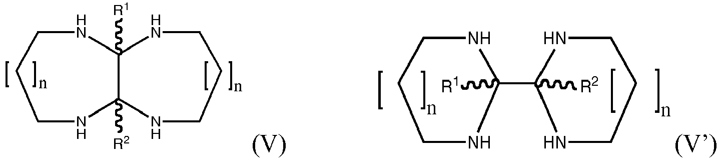 formule (V) et (V') dans lesquelles R¹ et R² sont tels que définis dans la formule (IV) ;
(ii) puis une étape au cours de laquelle on fait réagir l'intermédiaire obtenu avec un premier agent cyclisant,
(iii) et une étape au cours de laquelle on fait réagir le nouvel intermédiaire obtenu avec un deuxième agent cyclisant,
les étapes (ii) et (iii) pouvant être effectuées simultanément ou successivement, les agents cyclisant pouvant être identiques ou différents, et au moins l'un des deux agents cyclisants répondant à la formule (VI) telle que défini précédemment.

A titre d'agent cyclisant différent des agents cyclisants de formule (VI) utilisables, on peut citer n'importe quel agent cyclisant classique susceptible de cycliser les composés (V) et (V'), par exemple un composé Q-CH₂CH₂CH₂-Q' avec Q et Q', étant choisi parmi des groupes partants tels que des halogènes ou un groupe tosylate. De préférence, l'étape ou les étapes (i), (ii) et/ou (iii) ont lieu dans un milieu (ou solvant) protique. Plus préférentiellement, l'étape ou des étapes mettant en jeu un agent cyclisant de formule (VI) ont lieu dans un milieu protique.

Selon un mode de réalisation particulier, lorsque l'intermédiaire de formule (V) et/ou (V') est mis à réagir avec des agents cyclisants de formule (VI), identiques ou différents, un composé de formule (I') et/ou (II') est obtenu.

Le composé de formule (I') correspond à un composé de formule (I) dans laquelle :
A¹ ou A³ désigne un groupe C=O et :
- si A¹ désigne C=O et m est égal à zéro, alors A³ désigne CHR, ou si A³ désigne C=O et m est égal à zéro, alors A¹ désigne CHR ;
- ou si A³ désigne C=O et m est égal à un, alors A² ou A³ désigne CHR, ou si A¹ désigne C=O et m est égal à un, alors A² ou A³ désigne CHR ;
R répondant à l'une quelconque des définitions sus-indiquées.

Les composés de formule (I') sont illustrés par exemple par les composés de formule (Ia) à (If) décrits plus haut.

Les composés de formule (II') correspond à un composé de formule (II) dans laquelle :
A¹ ou A³ désigne un groupe C=O et :
- si A¹ désigne C=O et m est égal à zéro, alors A³ désigne CHR, ou si A³ désigne C=O et m est égal à zéro, alors A¹ désigne CHR ;
- ou si A³ désigne C=O et m est égal à un, alors A² ou A³ désigne CHR, ou si A¹ désigne C=O et m est égal à un, alors A² ou A³ désigne CHR ;
R répondant à l'une quelconque des définitions sus-indiquées.
Les composés de formule (II') sont illustrés par exemple par les composés de formule (IIa) à (IIf) décrits plus haut.

Selon un autre mode de réalisation particulier, lorsque l'intermédiaire de formule (V) est mis à réagir avec un agent cyclisant de formule (VI) et un agent cyclisant différent. De préférence, ce procédé permet d'obtenir un composé de formule (I") et/ou (II") tel que défini ci-dessous :
Le composé de formue (I") correspond à un composé de formule (I) dans laquelle :
- lorsque A¹ désigne C=O et m est égal à zéro, alors A³ désigne CH₂ ou C=O ; ou lorsque A³ désigne C=O et m est égal à zéro, alors A¹ désigne CH₂ ou C=O ;
- lorsque A³ désigne C=O et m est égal à un, alors A² et A³ sont choisis parmi les groupes CH₂ et C=O ; ou lorsque A¹ désigne C=O et m est égal à un, alors A² et A³ sont choisis parmi les groupes CH₂ et C=O.

Le composé de formule (II") correspond à un composé de formule (II) dans laquelle :
- lorsque A¹ désigne C=O et m est égal à zéro, alors A³ désigne CH₂ ou C=O ; ou lorsque A³ désigne C=O et m est égal à zéro, alors A¹ désigne CH₂ ou C=O ;
- lorsque A³ désigne C=O et m est égal à un, alors A² et A³ sont choisis parmi les groupes CH₂ et C=O ; ou lorsque A¹ désigne C=O et m est égal à un, alors A² et A³ sont choisis parmi les groupes CH₂ et C=O.

Lorsque dans la formule (VI), R⁵ forme avec R³ ou R⁴ un groupe hétérocyclique, ce groupe hétérocyclique répond de préférence à l'une des formules (X) ou (XI) suivantes: 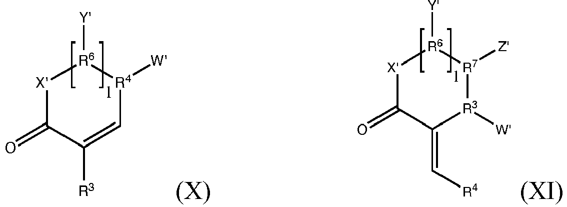
formule (X) dans laquelle : R³ est un groupe R tel que défini dans la formule (I) ou (II),
X' est un atome d'oxygène,
Y' et W', identiques ou différents, sont choisis parmi un atome d'hydrogène, un groupe alkyle, linéaire ou ramifié, saturé ou insaturé, en C₁-C₃₀,
1 est égal à zéro ou un, avec
   - lorsque 1 est égal à zéro, le groupe X' est relié au groupe R⁴ et R⁴ formant avec W' un groupe CH₂ ou C=O,
   - lorsque 1 est égal à un, R⁴ et R⁶ forment ensemble avec les groupes Y' et W' des groupes CH₂-CH₂, CH=CH, CH₂(C=O), (C=O)CH₂ ou un groupe phényle ;
      formule (XI) dans laquelle : R⁴ est un groupe R tel que défini dans la formule (I) ou (II), X' est un atome d'oxygène
W', Y' et Z', identiques ou différents, sont choisis parmi un atome d'hydrogène, un groupe alkyle, linéaire ou ramifié, saturé ou insaturé, en C₁-C₃₀,
R³, R⁶ et R⁷ désignant des groupes CH₂,
1 est égal à zéro ou un, et
   - lorsque 1 est égal à un,
      R³ et R⁷ peuvent former ensemble avec les groupes Z' et W', les groupes : CH₂-CH₂, CH=CH, ou un groupe phényle ; et/ou
      R⁶ et R⁷ peuvent former ensemble avec les groupes Y' et Z' les groupes : CH₂-CH₂, CH=CH, ou un groupe phényle ;
   - lorsque 1 est égal à zéro, le groupe X' est relié au groupe R⁷.

Dans les procédés décrits ci-dessus, les étapes (i), (ii) et/ou (iii) ont lieu de préférence dans un même milieu, plus préférentiellement dans un solvant protique. A titre d'exemples de solvants protiques utilisables, on peut citer l'eau, les alcools inférieurs en C₁-C₄, tels que le méthanol, l'éthanol, le propanol, le butanol.

Les procédés de préparation ainsi obtenus sont respectueux de l'environnement. Ils peuvent être réalisés en « one pot », c'est-à-dire *in situ* dans un même milieu réactionnel. En particulier, les étapes (i), (ii) et/ou (iii) peuvent se faire sans séparation ou sans purification intermédiaire.

Les étapes (i), (ii) et/ou (iii) peuvent être mises en oeuvre à des températures T1 et T2, inférieures ou égales à 100°C avec T1 étant inférieure à T2. De préférence, la température T1 est inférieure à 30°C, plus préférentiellement, elle varie de 0 à 25°C. La température T2 est choisie de préférence comme étant la température de reflux du solvant dans le milieu réactionnel. De préférence, elle est inférieure à 60°C, et mieux encore, elle est inférieure à 50°C.

Avantageusement, l'étape ou les étapes mettant en oeuvre le ou les agents cyclisants, notamment l'étape mettant en oeuvre l'agent cyclisant de formule (VI), peut être réalisée en appliquant des micro-ondes. Ceci permet d'augmenter la vitesse de réaction et donc de raccourcir la durée de réaction. La réaction peut par exemple être assistée par micro ondes.

L'invention porte également sur divers composés cycliques tétraazotés, dérivés du cyclam, C- fonctionnalisé(s) et/ou N-fonctionnalisé(s) pouvant être obtenus à partir d'un composé de formule (I), (II), un de leurs sels, solvates, ou mélange, tel que défini précédemment, ainsi que leurs procédés de préparation.

Le ou les procédés de préparation des composés cycliques tétraazotés, dérivés du cyclam, C- fonctionnalisé(s) et/ou N-fonctionnalisé(s) à partir d'un composé de formule (I), (II), un de leurs sels, solvates ou mélange, selon l'invention, comprennent au moins l'une des étapes suivantes :
A) substitution d'un ou plusieurs des quatre atomes d'azote du composé de formule (I) et/ou (II) par un ou plusieurs groupes R", identiques ou différents, de préférence identiques, R" pouvant désigner n'importe quel groupe fonctionnel ou précurseur de groupe fonctionnel, tel que défini précédemment, ou groupe protecteur de fonction amine, par exemple à l'aide d'au moins un agent électrophile R"Q, Q étant un groupe partant, tel qu'un atome d'halogène, un groupe tosylate, et/ou à l'aide d'au moins un réactif de Michael porteur du groupe R".
B) réduction d'une ou plusieurs fonctions cétone en position alpha d'un des quatre atomes d'azote du composé de formule (I) et/ou (II), par exemple par un ou plusieurs agents réducteurs, identiques ou différents, le(s)dit(s) composé(s) de formule (I) et/ou (II) ayant éventuellement été modifié(s) par une ou plusieurs étapes A) et/ou C).
C) déprotection du pont bisaminal du composé (I) et/ou (II), par exemple à l'aide d'un ou plusieurs agents réducteurs, identiques ou différents, le(s)dit(s) composé(s) de formule (I) et/ou (II) ayant éventuellement été modifié(s) par une ou plusieurs étapes A) et/ou B).

L'étape de déprotection C) peut être totale ou partielle, selon la nature de l'agent réducteur utilisé.

A titre d'exemple d'agent réducteur utilisable pour la déprotection partielle du pont bisaminal, on peut citer les agents réducteurs sélectifs, tels que le borohydrure de sodium (NaBH₄), l'hydrate d'hydrazine ou la soude. En particulier, on utilisera de préférence NaBH₄ pour obtenir une cis-réduction des amines du pont bisaminal. En particulier, on utilisera de préférence un hydrate d'hydrazine ou de la soude pour obtenir une trans-réduction des amines du pont bisaminal. Les schémas de préparation décrits plus loin permettront d'illustrer la régiosélectivité des étapes de déprotection partielle, selon la nature de l'agent réducteur utilisé.

A titre d'exemple d'agent réducteur utilisable pour la déprotection partielle du pont bisaminal, on peut citer les agents réducteurs forts tels que l'hydrure de lithium (LiAlH₄),ou BH₃ dans du tétrahydrofurane (THF).

Les étapes B) et C) peuvent être réalisées simultanément ou séparément, en utilisant par exemple un même agent réducteur ou différents agents réducteurs.

Lorsqu'au moins un des groupes R" cité dans l'étape A) désigne un groupe protecteur de fonction amine, par exemple un groupe benzyle, alors l'étape A) peut être suivie par une étape D) de déprotection de la fonction amine à l'aide d'un agent déprotecteur approprié. Par exemple, lorsque R" est un groupe benzyle, l'étape D) de déprotection peut être réalisée à l'aide d'une hydrogénolyse par exemple catalysé par du palladium sur charbon.

Lorsqu'au moins un des groupes R" cité dans l'étape A) désigne un groupe précurseur d'un groupe fonctionnel, alors l'étape A) peut être suivie par une étape ultérieure permettant de transformer R" en groupe fonctionnel, par exemple un traitement en milieu acide permettant de transformer un ester en acide carboxylique.

Selon une première variante, l'invention porte sur un procédé de préparation d'un composé C-fonctionnalisé de formule (XII) suivante, un de ses sels, solvates, ou mélange : 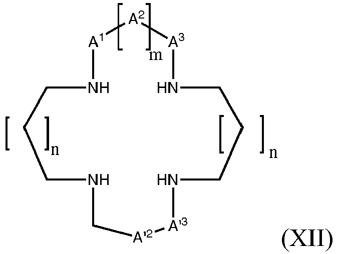 dans laquelle : A¹, A², A³, A'², A'³, m et n, sont tels que définis dans la formule (I) ou (II), ledit procédé comprenant une étape de réduction du composé de formule (I) ou (II), ou de leur mélange.

L'étape de réduction est réalisée à l'aide d'au moins un agent réducteur.

A titre d'agent réducteur utilisable dans ce procédé de préparation, on cite par exemple l'hydrure de lithium (LiAlH₄), les borohydrures tel que BH₃ dans du tétrahydrofuranne.

Selon une seconde variante, l'invention porte sur un procédé de préparation d'un composé C et N-tétra-fonctionnalisé de formule (XIII) suivante, un de ses sels, solvates, ou mélange : 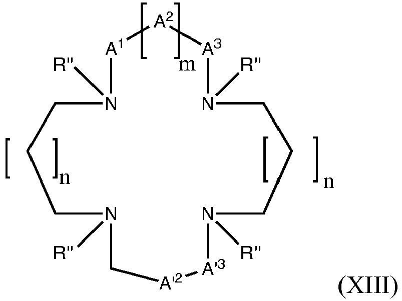 dans laquelle : A¹, A², A³, A'², A'³, m et n sont tels que définis dans la formule (I), ou (II), les groupes R", identiques ou différents, de préférence identiques, pouvant désigner n'importe quelle groupe fonctionnel, précurseur de groupe fonctionnel ou groupe protecteur, ledit procédé comprenant les étapes suivantes :
(i) réduction d'un composé de formule (I) ou (II), tel que défini précédemment, ou de leur mélange, à l'aide d'au moins un agent réducteur, et enfin
(ii) substitution, simultanée ou successive, sur les quatre atome d'azote du cycle tétraazoté du composé de formule (I) ou (II) réduit, ou (I) et (II) réduits en cas de mélange, par des groupes R", identiques ou différents, par exemple à l'aide d'au moins un agent électrophile R"Q, Q étant un groupe partant, tel qu'un atome d'halogène, un groupe tosylate, et/ou à l'aide d'au moins un réactif de Michael porteur du groupe R".

En particulier, le ou les groupes R" de la formule (XIII) peuvent désigner un groupe fonctionnel ou un précurseur d'un groupe fonctionnel, tels que définis précédemment, possédant de préférence au moins une fonction de coordination, et/ou un groupe protecteur d'un atome d'azote du composé cyclique tétraazoté.

Le ou les groupes R" peuvent être choisis parmi les groupes fonctionnels possédant au moins une fonction de coordination, par exemple un chélateur d'ions notamment d'ions métalliques, tels que acide, amine, alcool, imine, phosphonate, amide, 2,6-pyridine et ses isomères, tétrazole, dérivés souffrés tel que thiazole, propanoate, acétate, propanol, indazole.

Le ou les groupes R" peuvent également être des précurseurs de groupe fonctionnel, tel qu'un ester tertiobutylique, par exemple un acétate de tertiobutyle. L'ester tertiobutilique doit être traité en milieu acide (HCl) pour obtenir le groupe fonctionnel acide correspondant.

Le ou les groupes R" peuvent également être des groupes protecteurs de fonction amine du composé cyclique tétraazoté, tels que des groupes benzyle. Ces groupes servent notamment à induire ou favoriser la régiosélectivité d'une réaction ultérieure, et sont suffisamment inertes pour ne pas réagir lors de cette étape réactionnelle. Ces groupes peuvent ensuite être clivés par une réaction de déprotection adaptée. Lorsque le groupe R" est un groupe benzyle, il peut être déprotégé par hydrogénolyse notamment par une hydrogénolyse catalytique sur du palladium sur charbon.

A titre d'agent réducteur utilisable dans ce procédé de préparation, on cite par exemple l'hydrure de lithium (LiAlH4), les borohydrures tel que BH₃ dans du tétrahydrofuranne.

Selon une troisième variante, l'invention porte sur un procédé de préparation d'un composé C-fonctionnalisé et N-monofonctionnalisé de formule (XIV) suivante, un de ses sels, solvates ou mélange : 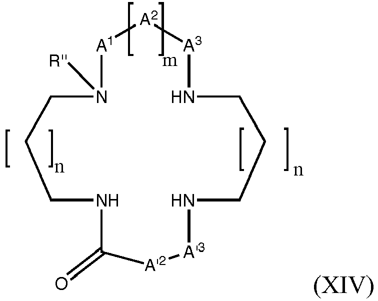 dans laquelle : A¹, A², A³, A'², A'³, m et n sont tels que définis dans la formule (I) ou (II), le groupe R" est tel que défini dans la formule (XIII), ledit procédé comprenant les étapes suivantes :
(i) substitution d'un atome d'azote d'un composé de formule (I) ou (II), ou de leur mélange, par un groupe protecteur R", par exemple à l'aide d'un agent électrophile R"Q, Q étant un groupe partant, tel défini que ci-dessus, puis
(ii) déprotection du pont bisaminal avec de l'hydrate d'hydrazine ou de la soude.

La substitution à l'étape (i) conduit notamment à la formation des intermédiaires cationiques (XIV-1) et/ou (XIV-2) suivants : 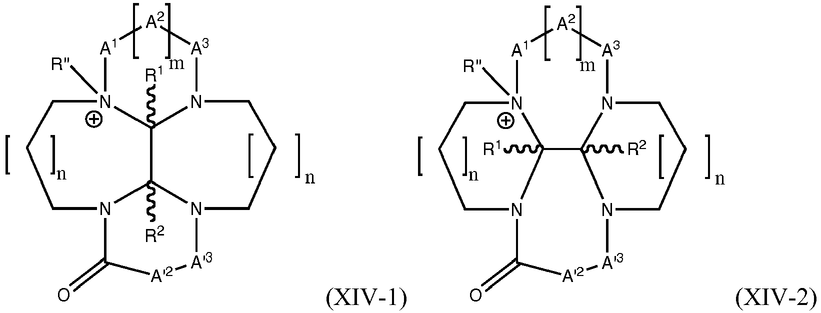 dans lesquelles : A¹, A², A³, A'², A'³, R¹ et R², m et n sont tels que définis dans la formule (I) ou (II) et R" tel que défini dans la formule (XIII).

Le composé (XIV) peut être réduit ultérieurement à l'aide d'un agent réducteur approprité, tel que BH₃ dans du THF, pour préparer un composé C-fonctionnalisé et N-monofonctionnalisé de formule (XIV'), un de ses sels, solvates ou mélange: 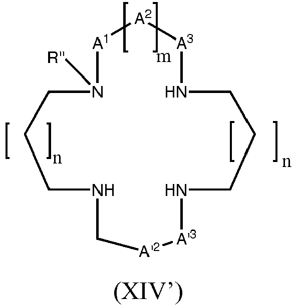 dans laquelle: A¹, A², A³, A'², A'³, R", m et n sont tels que définis dans la formule (XIV-1) ou (XIV-2).

Selon une quatrième variante, l'invention porte sur un procédé de préparation d'un composé C-fonctionnalisé et N-di-trans-fonctionnalisé de formule (XV) suivante, un de ses sels, solvates ou mélange: 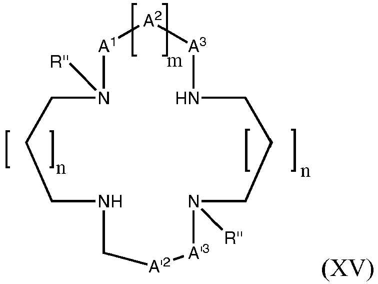 dans laquelle : A¹, A², A³, A'², A'³, m et n sont tels que définis dans la formule (I) ou (II), les groupes R", identiques ou différents, de préférence identiques, sont tels que définis dans la formule (XIII), ledit procédé comprenant les étapes suivantes :
(i) réduction d'un composé de formule (I) ou (II), tel que défini précédemment, ou de leur mélange, à l'aide d'un agent réducteur sélectif, puis
(ii) substitution sur deux atomes d'azote non adjacent du cycle tétraazoté du composé de formule (I) ou (II) réduit, ou (I) et (II) réduit en cas de mélange, par des groupes R", par exemple à l'aide d'au moins un agent électrophile R"Q, Q étant un groupe partant tel que défini ci-dessus, et enfin
(iii) déprotection du pont bisaminal avec de l'hydrate d'hydrazine ou de la soude.

A titre d'agent réducteur sélectif utilisable, on cite par exemple le borohydrure de sodium (NaBH₄). L'agent réducteur sélectif permet de réduire la fonction cétone du cycle tétraazoté (I) ou (II) sans déprotéger la fonction bisamine.

Par « atomes d'azote non adjacent du cycle », on entend que ces atomes d'azote constitutif du cycle tétraazacycloalcane sont séparés par un atome d'azote constitutif du cycle.

La substitution à l'étape (ii) conduit notamment à la formation des intermédiaires cationiques (XV-1) et/ou (XV-2) suivants: 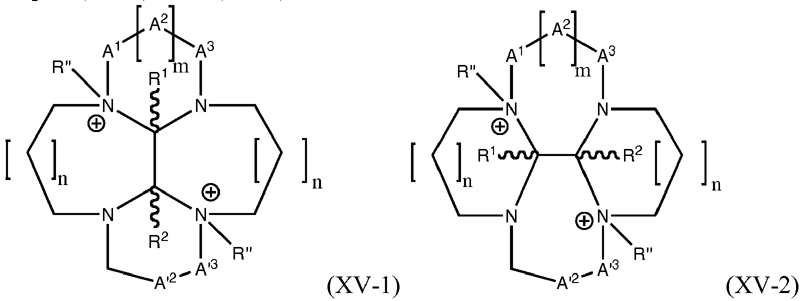 dans lesquelles : A¹, A², A³, A^{,2}, A'³, R¹ et R², m et n sont tels que définis dans la formule (I) ou (II).

Lorsque dans la formule (XV), R" désigne un groupe protecteur, tel qu'un groupe benzyle, le composé de formule (XV) peut être utilisé comme réactif de départ pour préparer un composé C-fonctionnalisé et N-di-trans-fonctionnalisé de formule (XV') suivante, un de ses sels, solvates ou mélange: 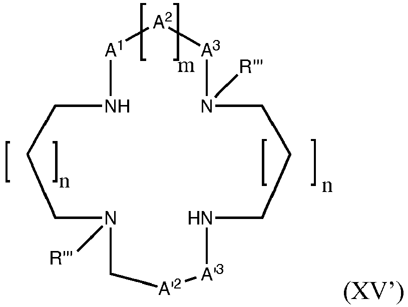 dans laquelle : A¹, A², A³, A'², A'³, m et n sont tels que définis dans la formule (I) ou (II), les groupes R'", identiques ou différents, de préférence identiques, correspondent à des groupes fonctionnels ou précurseurs de groupes fonctionnels R" tels que définis dans la formule (XIII).

Le composé (XV') peut être préparé en mettant en oeuvre les étapes (i) à (iii) comme décrit précédemment, par exemple, puis en mettant en oeuvre les étapes suivantes:
(iv) substitution des deux amines secondaires non substituées du cycle tétraazoté de formule (XV) par des groupes R'", tels que définis ci-dessus, par exemple à l'aide d'un agent électrophile R'"Q, Q étant un groupe partant tel que défini ci-dessus, puis
(v) déprotection des groupes protecteurs R".

Lorsque R" est un groupe benzyle, l'étape (v) de déprotection peut être réalisée à l'aide d'une hydrogénolyse par exemple, catalysée par du palladium sur charbon.

Selon une cinquième variante, l'invention porte sur un procédé de préparation d'un composé dit « cross bridge » C-fonctionnalisé et N-di-trans-fonctionnalisé de formule (XVI) suivante, un de ses sels, solvates, ou mélange : 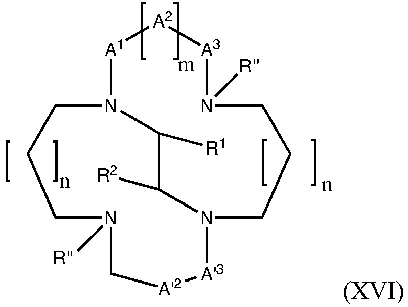 dans laquelle : A¹, A², A³, A^{,2}, A'³, R¹ et R², m et n sont tels que définis dans la formule (I) ou (II), les groupes R", identiques ou différents, de préférence identiques, sont tels que définis dans la formule (XIII), ledit procédé comprenant les étapes suivantes:
(i) réduction d'un composé de formule (I) ou (II) tel que défini précédemment, ou leur mélange, à l'aide d'un agent réducteur sélectif, puis
(ii) substitution des amines secondaires du cycle (I) ou (II) réduit, ou des cycles (I) et (II) réduits en cas de mélange, par des groupes R", tels que définis ci-dessus, par exemple à l'aide d'agent électrophile R"Q, Q étant un groupe partant tel que défini ci-dessus, et enfin
(iii) réduction partielle du pont bisaminal à l'aide d'un agent réducteur sélectif.

A titre d'agent réducteur sélectif utilisable aux étapes (i) et (iii), on cite par exemple le borohydrure de sodium (NaBH₄).
Lors de l'étape (i), l'agent réducteur sélectif permet de réduire la fonction cétone du cycle tétraazoté (I) ou (II) sans déprotéger la fonction bisamine.
Lors de l'étape (iii), l'agent réducteur sélectif permet de réduire partiellement le pont bisaminal.

La substitution à l'étape (ii) conduit notamment à la formation des intermédiaires cationiques XVI-1 et/ou (XVI-2) suivants: 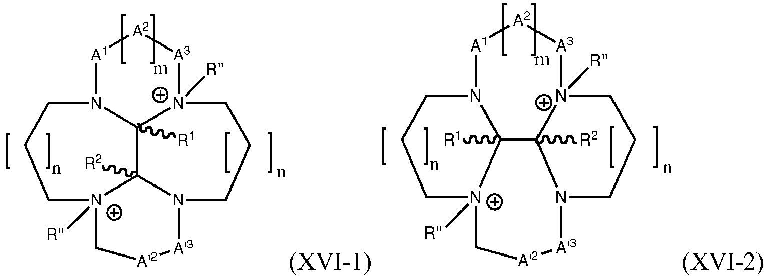 dans lesquelles : A¹, A², A³, A^{,2}, A'³, R¹ et R², m et n sont tels que définis dans la formule (I) ou (II), R" tels que définis dans la formule (XIII).

Lorsque dans la formule (XVI), R" est un groupe protecteur tel que benzyle, il peut être déprotégé après l'étape (iii) à l'aide d'une hydrogénolyse par exemple, catalysée par du palladium sur charbon.

Selon une sixième variante, l'invention porte sur un procédé de préparation d'un composé dit « side bridge » N- mono fonctionnalisé de formule (XVII) suivante, un de ses sels ou solvates, ou leur mélange: 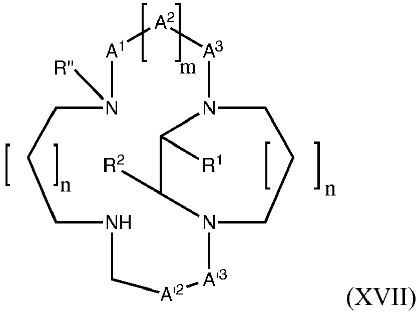 formule (XVII) dans laquelle : A¹, A², A³, A'², A'³, R¹ et R², m et n sont tels que définis dans la formule (I) ou (II), le groupe R" est tel que défini dans la formule (XIII), ledit procédé comprenant les étapes suivantes:
(i) substitution d'une amine secondaire du composé de formule (I) et/ou (II) , par un groupe R" tel que défini dans la formule (XIII), par exemple à l'aide d'un agent électrophile R"Q, Q étant un groupe partant tel que défini ci-dessus, puis
(ii) réduction partielle du pont bisaminal à l'aide d'un agent réducteur sélectif, tel que NaBH₄.

La substitution à l'étape (i) conduit notamment à la formation des intermédiaires cationiques (XVII-1) et/ou (XVII-2) suivants : 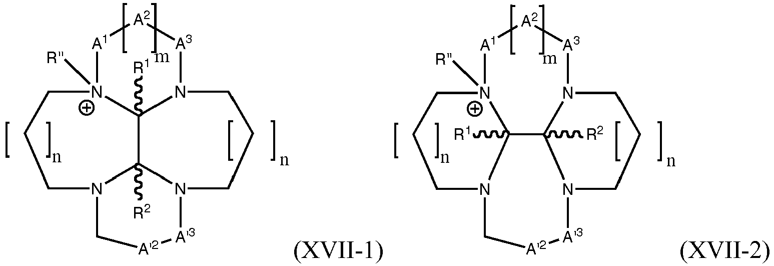 dans lesquelles : A¹, A², A³, A^{,2}, A'³, R¹ et R², m et n sont tels que définis dans la formule (I) ou (II), les groupes R", identiques ou différents, sont tels que définis dans la formule (XIII).

Le composé de formule (XVII) peut être utilisé comme réactif de départ pour préparer un composé dit « side bridge » C-fonctionnalisé et N-di-fonctionnalisé de formule (XVII'), suivante, un de ses sels ou solvates, ou leur mélange: 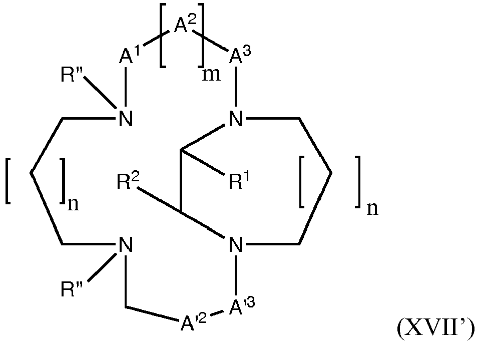 dans laquelle : A¹, A², A³, A^{,2}, A'³, R¹ et R², m et n sont tels que définis dans la formule (I) ou (II), les groupes R", identiques ou différents, sont tels que définis dans la formule (XIII).

Le composé (XVII') peut être préparé en mettant en oeuvre les étapes (i) et (ii) comme décrit précédemment par exemple, puis en mettant en oeuvre l'étape (iii) suivante:
(iii) substitution de l'amine secondaire non substituée du cycle tétraazoté du composé de formule (XVII) par un groupe R" tel que défini dans la formule (XIII), par exemple à l'aide d'un agent électrophile R"Q, Q étant un groupe partant tel que défini précédemment.

Par « side bridge », on entend que le composé cyclique possède une structure pontée (pont éthylène) entre deux atomes d'azote adjacents constitutifs du cycle tétraazoté.

Par « atomes d'azote adjacents constitutifs du cycle tétraazoté », on entend que les deux atomes d'azotes sont reliés par une chaîne d'atomes de carbone constitutifs du cycle tétraazoté, non interrompue pas un atome d'azote.

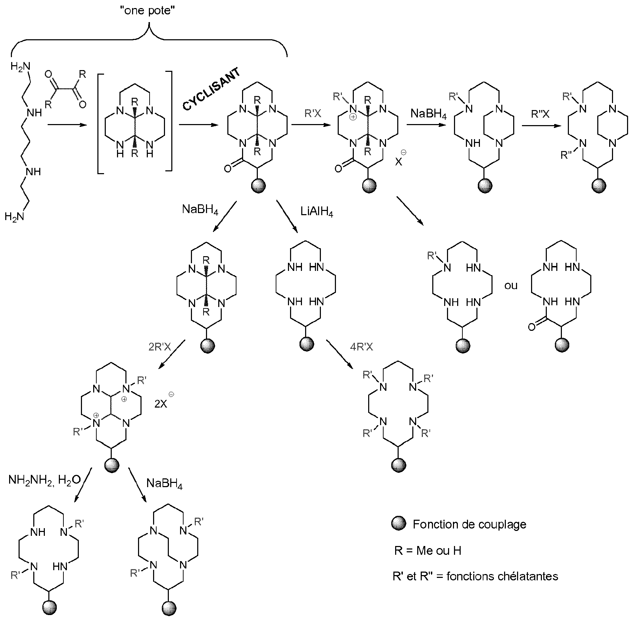

La figure 1 donne un aperçu de quelques uns des différents procédés selon l'invention.

Dans toutes ces variantes énumérées ci-dessus, le composé de formule (I) et /ou (II) mis à réagir peut être obtenu à l'aide d'un des deux procédés de préparation principaux selon l'invention précédemment décrits précédemment, c'est-à-dire soit à partir de deux diamines, soit à partir d'une tétramine, telles que définies plus haut.

Les composés cycliques bisaminals C-fonctionnalisés selon l'invention et les composés obtenus à partir de tels composés, comme décrits précédemment, peuvent être sous forme de sels ou de solvates.

Les sels peuvent être choisis parmi les sels formés avec un acide inorganique, comme l'acide chlorhydrique, sulfurique, phosphorique, nitrique, carbonique, borique, sulfamique, bromhydrique ; ou bien les sels formés avec un acide organique, comme l'acide acétique, propionique, butyrique, tartarique, maléique, hydroxymaléique, fumarique, maléique, citrique, lactique, mucique, gluconique, benzoïque, succinique, oxalique, phénylacétique, méthanesulfonique, toluènesulfonique, benzènesulfonique, salicylique, sulfanilique, aspartique, glutamique, édétique, stéarique, palmitique, oléique, laurique, panthoténique, tannique, ascorbique et valérique. Lorsque le composé selon l'invention comporte une fonction acide, il peut se présenter sous la forme d'un sel avec une base organique ou minérale. Hormis les cas où R¹ et R² des formules précédentes réprésentent tous les deux des atomes d'hydrogène, le ou les éventuels sels de ces composés sont de préférence des sels formés avec une base organique ou minérale.

Parmi tous les composés C-fonctionnalisés et N-fonctionnalisés décrits ci-dessus, on préfère ceux pour lesquels R représente un groupe fonctionnel ayant une fonction de couplage, par exemple alcool, et R" ou R'" représente un groupe fonctionnel ayant une fonction de coordination.

Grâce à leur propriété chélatante et leur capacité à être greffé sur un support donné, les composés obtenus à partir des composés cycliques bisaminals C-fonctionnalisé selon l'invention peuvent être utilisés dans divers domaines d'application tels que la catalyse, le magnétisme, les matériaux, l'épuration de liquides et de gaz, les méthodes d'analyse et de détection, la médecine, notamment la médecine nucléaire, en particulier comme agent d'imagerie par tomographie par émission de positons (TEP) (émetteur P⁺ : ⁶⁴Cu, ⁶⁸Ga...) ou pour la radioimmunothérapie (RIT) (émetteur α, β⁺ ou β⁻ : ^{212, 213}Bi, ⁶⁴Cu, ⁶⁷Cu,...), notamment vis-à-vis des cellules cancéreuses, telles que les mélanomes, neuroblastes, cellules cancéreuses de la prostate ou de l'estomac.

Le composé cyclique bisaminal C-fonctionnalisé selon l'invention peut ainsi être utilisé pour la fabrication d'un agent chélateur métallique et/ou d'un radionucléide (cuivre, bismuth ou gallium).

En particulier, le composé de formule (I) ou (II), plus particulièrment le composé de formule (III), peut être utilisé pour fabriquer un radiopharmaceutique (radioimmunothérapie (RIT)) ou un agent d'imagerie par tomographie par émission de positons (TEP), en particulier, tel que défini ci-dessus.

Les exemples ci-dessous servent à illustrer les différents objets de l'invention.

### EXEMPLES :

### 1) Instrumentation

### Spectrométrie de Résonance Magnétique Nucléaire (RMN)

Les spectres RMN ont été enregistrés à l'aide des appareillages suivants :
- BRUKER AMX 3 300 (¹H : 300,13 MHz ; ¹³C : 75,47 MHz)
- BRUKER DRX 500 (¹H : 500,13 MHz ; ¹³C : 125,76 MHz)

Les spectres sont référencés à l'aide de solvant deutéré. Les déplacements chimiques sont indiqués en ppm.

### Spectroscopie Infrarouge (IR)

Le spectre Infrarouge a été réalisé sur un appareil Perkin Elmer (Spectrum One).

### Spectrométrie de masse (MS)

Les mesures des masses moléculaires ont été effectuées à l'aide d'un spectromètre Autoflex MALDI TOF III LRF200 CID en utilisant le dithranol ou l'acide 2,5-dihydroxybenzoïque (DHB) comme matrice.

### Exemple 1 : Préparation des cyclams-glyoxal-amide C-fonctionnalisé (1) et (2)

### 1) Cyclam-cis-glyoxal-amide C-CH₂CH₂OH (1)

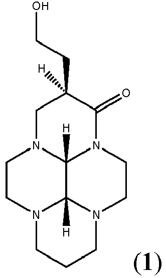

40 mL d'une solution méthanolique de glyoxal à 40 % en masse (31,20 mmol ; 1,81 g) sont ajoutés, goutte à goutte, à une solution de 1,4,8,11-tétraazatétradécane (31,20 mmol ; 5,00 g) dans 100 mL de méthanol à - 5 °C. L'agitation est maintenue pendant 2 heures. Après retour à température ambiante (25°C), une solution de α-méthylène-γ-butyrolactone (31,20 mmol; 3,50 g) dans 40 mL de méthanol est ajoutée, goutte à goutte, au milieu réactionnel. La solution est agitée à 45 °C pendant 7 jours. Après évaporation du solvant, le mélange est recristallisé dans l'acétronile. Le composé **(1)** est obtenu sous forme de cristaux blancs lavés avec de l'éther diéthylique (C₁₄H₂₄N₄O₂, M= 280,19 g.mol⁻¹ ; 15,60 mmol ; 4,37 g; rendement 50 %).
**RMN ¹H (300 MHz, 298K, CDCl₃, *δ* en ppm) :** 5,50 (s, 1H, **OH**) ; 4,45 (d, *J*=6,8 Hz, 1H, C**H**₂-α-N) ; 4,31 (s, 1H, **CH**) ; 3,38 (m, 1H, C**H**₂-OH) ; 3,36 (t, *J*=6,8 Hz, 1H, C**H**₂-OH) ; 3,35 (t, *J*=6,8 Hz, 1H, C**H**₂-α-N) ; 3,18 (s, 1H, C**H**) ; 3,09 (m, 2H, C**H**₂-α-N) ; 2,96 (m, 4H, C**H**₂-α-N) ; 2,76 (m, 3H, C**H**₂-α-N, CH-β-N) ; 2,53 (d, *J*=6,8 Hz, 1H, C**H**₂-α-N) ; 2,44 (d, *J*=6,8 Hz, 1H, C**H**₂-α-N) ; 2,25 (m, 1H, C**H**₂-α-N) ; 2,15 (m, 2H, C**H**₂-α-N, CH₂-β-N) ; 1,92 (m, 1H, C**H**₂-γ-N) ; 1,37 (d, *J*=8,5 Hz, 1H, C**H**₂-γ-N) ; 1,27 (d, *J*=8,5 Hz, 1H, C**H**₂-β-N).
**RMN ¹³C (300 MHz, 298K, CDCl₃, *δ* en ppm)** : 173,1 (CO) ; 75,7 (**C**H) ; 70,6 (**C**H) ; 61,7 (**C**H₂-OH); 55,6 (**C**H₂-α-N); 54,9 (**C**H₂-α-N); 53,0 (**C**H₂-α-N); 52,7 (**C**H₂-α-N); 44,1 (**C**H₂-α-N) ; 43,6 (**C**H₂-α-N) ; 40,3 (**C**H₂-α-N) ; 35,4 (**C**H-β-N) ; 31,7 (**C**H₂-γ-N) ; 19,3 (**C**H₂-β-N).
**IR** : υ_{amide} = 1616 cm⁻¹.
**Analyse élémentaire** : Calculée = C 59,98; H 8,63; N 19,98. Trouvée = C 59,91; H 8,71; N 20,09.
**Spectrométrie de masse (MALDI-TOF) :** m/z = 281,23 g.mol⁻¹ (M+1)⁺

### 2) Cyclam-cis-glyoxal-amide C-CH₂COOCH₃ (2)

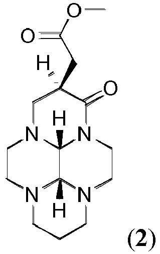

40 mL d'une solution méthanolique de glyoxal à 40 % en masse (24,96 mmol ; 1,40 g) sont ajoutés, goutte à goutte, à une solution de 1,4,8,11-tétraazatétradécane (24,96 mmol ; 4,00 g) dans 100 mL de méthanol à -5 °C. L'agitation est maintenue pendant 2 heures. Après retour à température ambiante (25°C), une solution de diméthylitaconate (24,96 mmol ; 3,50 g) dans 40 mL de méthanol est ajoutée, goutte à goutte, au milieu réactionnel. La solution est agitée à 45 °C pendant 14 jours. Le mélange est porté à sec puis purifié par chromatographie sur gel de silice avec comme éluant un mélange CHCl₃/CH₃OH (en proportions 100/0-94/6 en volume par volume (v/v)). Le composé (2) se présente sous la forme d'une huile jaune (**C₁₅H₂₄N₄O₃**, M= 308,18 gmol⁻¹ ; 14,73 mmol ; 4,54 g ; rendement 59 %).
**RMN ¹³C (300 MHz, 298K, CDCl₃, *δ* en ppm) :** 172,5 (CO-amide) ; 170.8 (CO-ester) ; 76,0 (**C**H) ; 71,0 (**C**H) ; [55,9 ; 53,9 ; 53,2 ; 53,0 ; 44,5 ; 43,9 ; 40,5] (CH₂-α-N) ; 51,7 (-OCH₃) ; [33,2 ; 32,5] (CH-β-N ; C**H**₂-γ-N) ; 19,6 (CH₂-β-N).
**IR :** υ_{amide} = 1616 cm⁻¹.
**Analyse élémentaire** : Calculée = C 58,42; H 7,84; N 18,17. Trouvée = C 58,71; H 8,01; N 18,19.
**Spectrométrie de masse (MALDI-TOF)** : m/z= 309,21 gmol⁻¹ (M+1)⁺

### Exemple 2 : Synthèse d'un cyclam N-di-trans-fonctionnalisé C-fonctionnalisé (8)

### 1) Cyclam-cis-glyoxal C-CH₂CH₂OH (3)

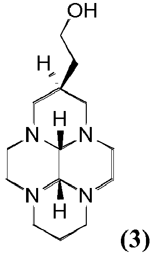

A une solution aqueuse du composé **(1)** (3,00g; 10,71 mmol) est ajouté lentement du borohydrure de sodium (107,10 mmol ; 4,05 g), à température ambiante. Le mélange est agité pendant 18 heures. Après évaporation du solvant, les sels de bore sont précipités par ajout de dichlorométhane. Le mélange est filtré et le solvant évaporé pour obtenir le composé **(3)** sous forme d'une huile jaune **(C₁₄H₂₆N₄O,** M= 266,38 gmol⁻¹ 2,71 g ; 10,18 mmol ; rendement 95 %).
**RMN ¹H (300 MHz, 298K, CDCl₃, *δ* en ppm)** : 3,64 (m, 2H, C**H**₂-OH) ; 3,51 (dt, J=20 Hz, *J*=3,4 Hz, 2H, C**H**₂-α-N) ; 3,10 (s, 1H, C**H**) ; 2,99 (m, 2H, C**H**, C**H**₂-α-N) ; 2,94 (m, 4H, C**H**₂-α-N) ; 2,78 (d, *J*=3,4 Hz, 1H, O**H**) ; 2,73 (m, 2H, C**H**₂-α-N) ; 2,57 (t, *J*=3,4 Hz, 2H, C**H**₂-α-N) ; 2,32 (m, 3H, C**H**₂-α-N, C**H**₂-γ-N) ; 2,22 (m, 2H, C**H**₂-α-N, C**H**₂-β-N); 2,14 (m, 1H, C**H**₂-α-N) ; 1,70 (m, 1H, C**H**₂-α-N) ; 1,34 (q, *J*=3,4 Hz, 2H, C**H**-β-N, C**H**₂-γ-N) ; 1,22 (m, 1H, C**H**₂-β-N).
**RMN ¹³C (300 MHz, 298K, CDCl₃, *δ* en ppm) :** 77,3 (**C**H × 2) ; 62,3 (**C**H₂-α-N) ; 60,4 (**C**H₂-OH) ; 58,8 (**C**H₂-α-N) ; 56,1 (**C**H₂-α-N × 2) ; 54,5 (**C**H₂-α-N) ; 52,6 (**C**H₂-α-N) ; 45,8 (**C**H₂-α-N) ; 44,8 (*C*H₂-α-N) ; 34,9 (**C**H-β-N) ; 28,2 (**C**H₂-γ-N) ; 19,7 (**C**H₂-β-N).
**Analyse élémentaire** : Calculée = C 62,12; H 9.84; N 21,03. Trouvée = C 61,89; H 9,92; N 21,19.
**Spectrométrie de masse (MALDI-TOF)** : m/z= 267,41 gmol⁻¹ (M+1)^{+.}

### 2) Cyclam-cis-glyoxal N-trans-dibenzyle C-CH₂CH₂OH (4)

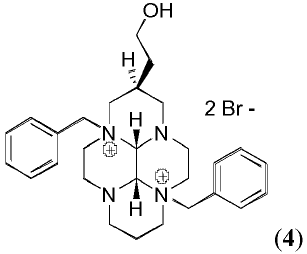

Une solution de bromure de benzyle (13,99 mmol ; 2,38 g) dans 20 mL d'acétonitrile distillé est ajoutée goutte à goutte à une solution du dérivé **(3)** (1,38 g ; 5,18 mmol) dans 45 mL d'acétonitrile distillé. Le mélange est agité à température ambiante pendant 7 jours. La solution est filtrée pour obtenir le composé **(4)** sous forme d'une une poudre blanche **(C₂₈H₄₀Br₂N₄O**, M= 606,16 gmol⁻¹ ; 1,88 g, rendement 60 %).
**RMN ¹H (300 MHz, 298K, CDCl₃, *δ* en ppm)** : 7,63 (m, 10H, **H-**Ar) ; 5,33 (t, *J*=7,7 Hz, 2H, C**H**₂-Ar) ; 5,13 (d, *J*=7,7 Hz, 2H, C**H**) ; 4,78 (m, 2H, C**H**₂-Ar) ; 4,46 (q, *J*=7,7 Hz, 2H, C**H**₂-α-N) ; 3,78 (t, *J*=7,7 Hz, 1H, C**H**₂-α-N) ; 3,64 (m, 4H, OH, C**H**₂-α-N, C*H*₂-OH) ; 3,50 (m, 5H, C**H**₂-α-N) ; 3,32 (m, 4H, C**H**₂-α-N) ; 2,87 (t, *J*=7,7 Hz, 1H, C**H**₂-α-N) ; 2,60 (m, 2H, C**H**₂-α-N, C*H*-β-N) ; 2,33 (m, 1H, C**H₂**-β-N) ; 2,29 (m, 1H, C**H**₂-γ-N) ; 1,95 (d, *J*=7,7 Hz, 2H, C**H₂**-β-N, C*H*₂-γ-N) ; 1,56 (m, 1H, C**H₂**-α-N).
**RMN ¹³C (300 MHz, 298K, CDCl₃, *δ* en ppm)** : 136,1 (**C**H-Ar × 4) ; 134,4 (**C**H-Ar × 4) ; 132,4 (**C**H-Ar × 2) ; 127,6 (**C**-Ar × 2) ; 79,8 (**C**H) ; 79,5 (**C**H) ; 67,3 (**C**H₂-α-N) ; 65,3 (**C**H₂-α-N) ; 65,3 (**C**H₂-α-N) ; 63,5 (**C**H₂-α-N) ; 61,2 (**C**H₂-OH) ; 59,7 (**C**H₂-α-N) ; 54,1 (**C**H₂-α-N) ; 49,7 (**C**H₂-α-N) ; 49,6 (**C**H₂-α-N × 2) ; 49,0 (**C**H₂-α-N) ; 35,0 (**C**H₂-γ-N) ; 28,6 (**C**H-β-N) ; 20,9 (**C**H₂-β-N).
**Analyse élémentaire** : Calculée = C 53,68; H 6.76; N 8,94. Trouvée = C 53,79; H 6.81; N 9,21 (C₂₈H₄₀Br₂N₄O, H₂O)
**Spectrométrie de masse (MALDI-TOF)** : m/z= 448,33 gmol⁻¹ (M)^{+.}

### 5) Cyclam N-trans-dibenzyle C-CH₂CH₂OH (5)

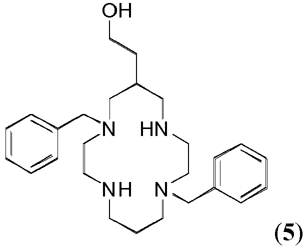

Le composé **(4)** (1,20 g; 1,98 mmol) est dissous dans 6,0 mL d'hydrate d'hydrazine. Le mélange est porté à reflux pendant 4 heures. Après refroidissement du mélange, l'hydrate d'hydrazine est éliminée. Après ajout de 15 mL d'eau distillée, le produit est extrait au chloroforme (3 x 30 mL). Les phases organiques recueillies, sont séchées sur du sulfate de magnésium, filtrées puis évaporées. Le composé **(5)** obtenu est une huile jaune **(C₂₆H₄₀N₄O,** M= 424,32 gmol⁻¹ ; 760 mg, 1,79 mmol ; rendement 90 %).
**RMN ¹H (300 MHz, 298K, CDCl₃, δ en ppm)** : 7,25 (m, 10H, **H**-Ar) ; 3,94 (d, *J*=7,9 Hz, 1H, C**H**₂-α-N) ; 3,72 (d, *J*=7,9 Hz, 2H, C**H₂**-Ar) ; 3,62 (m, 5H, C**H₂**-Ar, C*H*₂-OH, O**H**) ; 3,54 (m, 1H, C**H**₂-γ-N) ; 3,35 (d, *J*=7,9 Hz, 1H, C**H**₂-α-N) ; 2,92 (m, 1H, C**H**₂-α-N) ; 2,85 (m, 3H, C**H₂**-α-N) ; 2,65 (m, 3H, C**H**₂-α-N) ; 2,53 (m, 3H, C**H**₂-α-N) ; 2,45 (m, 1H, C**H₂**-α-N) ; 2,31 (d, *J*=7,9 Hz, 1H, C**H₂**-α-N) ; 2,16 (s, 3H, C**H**-β-N, N**H**) ; 2,11 (d, *J*=7,9 Hz, 1H, C**H₂**-α-N) ; 1,83 (m, 1H, C**H**₂-β-N); 1,72 (m, 1H, C**H**₂-β-N); 1,63 (m, 1H, C**H₂**-α-N); 1,50 (m, 1H, C**H**₂-γ-N).
**RMN ¹³C (300 MHz, 298K, CDCl₃, δ en ppm) :** 137,6 (C-Ar × 2) ; 129,4 (CH-Ar × 4) ; 128,3 (CH-Ar × 4) ; 127,2 (CH-Ar × 2) ; 59,1 (*C*H₂-OH, *C*H₂-α-N) ; 57,9 (*C*H₂-α-N × 2) ; 54,0 (*C*H₂-α-N) ; 52,5 (*C*H₂-α-N) ; 51,7 (*C*H₂-α-N) ; 51,2 (*C*H₂-α-N) ; 49,4 (*C*H₂-α-N) ; 47,8 (*C*H₂-α-N) ; 46,9 (*C*H₂-α-N) ; 36,4 (*C*H₂-γ-N) ; 31,0 (CH-β-N) ; 26,3 (*C*H₂-β-N)
**Analyse élémentaire** : Calculée = C 73,54; H 9.49; N 13,19. Trouvée = C 73,69; H 9,19; N 13,02.
**Spectrométrie de masse (MALDI-TOF) :** m/z= 425,32 gmol⁻¹ (M+1)⁺

### 6) Cyclam N-trans-dibenzyle N-trans-diacétate-tertiobutyle C-CH₂CH₂OH (6)

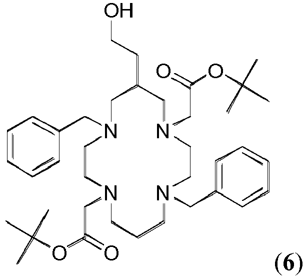

Une solution de bromoacétate de tertiobutyle (2,02 g ; 10,36 mmol) dans 45 mL d'acétonitrile distillé est ajoutée, goutte à goutte, à une solution du composé **(5)** (2,00 g ; 4,71 mmol) et de carbonate de potassium (2,60 g ; 18,84 mmol) dans 80 mL d'acétonitrile distillé, préalablement chauffée à 40 °C. La solution est agitée pendant 6 jours à 40 °C puis filtrée sur célite. Après évaporation du solvant, l'huile marron obtenue, est solubilisée dans 15 mL d'eau distillée. Une solution d'acide chlorhydrique 3M (mol/L) est ajoutée jusqu'à pH = 2. Après extraction à l'hexane des impuretés (5 x 30 mL), la phase aqueuse est basifiée jusqu'à pH = 12 par addition d'une solution de soude 4M. Le produit est extrait au chloroforme (5 x 60 mL) ; les phases oraganiques recueillies, sont séchées sur du sulfate de magnésium puis évaporées pour donner le composé **(6)** sous la forme d'une huile jaune **(C₃₈H₆₀N₄O₅,** M= 652,46 ; 1,94 g, 2,97 mmol, rendement 63 %).
**RMN ¹³C (300 MHz, 298K, CDCl₃, δ en ppm) :** [170,6 ; 170,4] (CO) ; [139,5 ; 138,2 ; 129,7 ; 128,9 ; 128,3 ; 128,1 ; 127,2 ; 126,8] (CH-Ar, C-Ar ×) ; [80,7 ; 80,6] (**C**(CH₃)₃) ; [61,3 ; 60,9 ; 60,8 ; 60,7 ; 60,0 ; 59,2 ; 57,4 ; 56,7 ; 51,6 ; 51,1 ; 51,0 ; 50,8 ; 48,9] (**C**H₂-OH, CH₂-α-N) ; [37,8 ; 35,7] (CH-β-N, C**H**₂-γ-N) ; 28,0 (C(CH₃)₃ × 2) ; 24,9 (CH₂-β-N).
**Spectrométrie de masse (MALDI-TOF) :** m/z= 653,49 gmol⁻¹ (M+1)^{+.}

### 7) Cyclam N-trans-diacétate tertiobutyle C-CH₂CH₂OH (7)

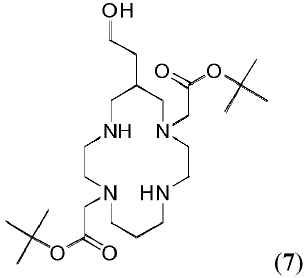

Le composé **(6)** (930 mg ; 1,43 mmol) est dissous dans 60 mL d'une solution de 200 mg de Palladium sur charbon activé (Pd/C activé) à 10 % en masse dans l'acide acétique glacial, sous atmosphère de dihydrogène. Le mélange est agité pendant 4 jours. Le produit obtenu se présente sous la forme d'une huile marron **(C₂₄H₄₈N₄O₅,** M= 472,36 gmol⁻¹ ; 392 mg, 0,83 mmol ; rendement 58 %).
**RMN ¹³C (300 MHz, 298K, CDClk₃, δ en ppm) :** [170,4 ; 170,3] (**C**O) ; 80,6 (**C**(CH₃)₃ × 2) ; [59,3 ; 57,8 ; 55,8 ; 54,7 ; 54,3 ; 53,4 ; 52,7 ; 52,4 ; 49,3 ; 47,6 ; 46,7] (*C*H₂-OH, **C**H₂-α-N) ; [35,9 ; 34,1] (**C**H-β-N, **C**H₂-α-N) ; 28,0 (C(**C**H₃)₃× ₂) ; 26,1 (**C**H₂-β-N). **Spectrométrie de masse (MALDI-TOF) :** m/z= 472,40 gmol⁻¹ (M+1)⁺

### 8) Cyclam N-trans-diacétate C-CH₂CH₂OH, 4.CF₃COOH (8)

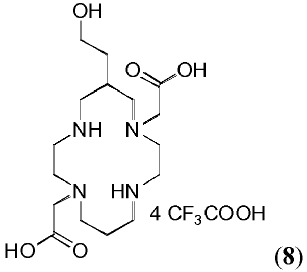

Le composé (7) (392 mg ; 0,83 mmol) est dissous dans 10 mL d'acide trifluoroacétique. La solution est agitée pendant 18 heures à température ambiante. Le solvant est évaporé. Le composé est obtenu quantitativement sous la forme d'un solide marron **(C₂₄H₃₆F₁₂N₄O₁₃,** M= 816,54 gmol⁻¹ ; 678 mg, 0,83 mmol ; quantitatif).
**RMN ¹³C (300 MHz, 298K, D₂O, δ en ppm) :** [178,1 ; 177,0] (CO) ; [67,6 ; 65,4 ; 60,6 ; 59,3 ; 58,3 ; 56,8 ; 55,3 ; 51,9 ; 51,3 ; 50,7 ; 47,5] (**C**H₂-OH, **C**H₂-α-N) ; [34,9 ; 32,4] (**C**H-β-N, CH₂-α-N) ; 24,6 (**C**H₂-β-N).
**Spectrométrie de masse (MALDI-TOF) :** m/z= 360,51 gmol⁻¹ (M+1)⁺

### Exemple 3 : Synthèse d'un cyclam-« cross bridge » C-fonctionnalisé (10)

### 1) Cyclam « cross bridge » N-trans-dibenzyle C-CH₂CH₂OH (9)

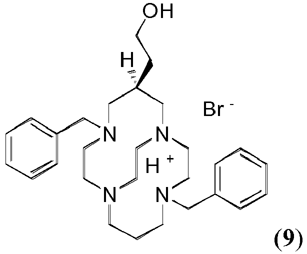

Du borohydrure de sodium (998 mg ; 26,40 mmol) est ajouté lentement à 50 mL d'une solution éthanolique à 95 % en masse du composé **(4)** (1,00 g ; 1,65 mmol). Le mélange est agité pendant 12 jours à température ambiante (25°C). Après évaporation du solvant, les sels de bore sont précipités par ajout de dichlorométhane. Le mélange est filtré et le solvant évaporé pour obtenir le composé sous forme d'une huile jaune (C₂₈H₄₃BrN₄O, M=530,26 g.mol⁻¹ ; 744 mg ; 1,40 mmol ; rendement 85 %).
**RMN ¹H (300 MHz, 298K, CDCl₃, δ en ppm) :** 10,29 (s, 1H, N**H**⁺) ; 7,26 (m, 10H, **H**-Ar) ; 3,73 (m, 5H, C**H**₂-α-N, C**H₂**-Ar × 2) ; 3,66 (m, 4H, O**H,** C**H**₂-α-N, C**H₂**-OH ) ; 3,49 (d, *J*=7,1 Hz, 5H, C**H₂**-α-N × 4) ; 3,31 (m, 2H, C**H₂**-α-N × 2) ; 3,13 (m, 3H, C**H₂**-α-N × 3) ; 3,00 (m, 3H, C**H₂**-α-N × 2) ; 2,76 (m, 4H, C**H₂**-α-N × 3, C**H**-β-N) ; 2,42 (m, 2H, C**H**₂-α-N, C*H*₂-γ-N) ; 1,96 (s, 1H, C**H₂**-β-N) ; 1,59 (m, 1H, C**H₂**-α-N) ; 1,36 (m, 1H, C**H**₂-γ-N) ; 1,18 (m, 1H, C**H**₂-β-N).
**RMN ¹³C (300 MHz, 325K, CDCl₃)** : δ (ppm) [136,1 ; 134,1 ; 130,2 ; 129,7 ; 128,4 ; 128,3 ; 127,6 ; 127,4] (CH-Ar, C-Ar ×) ; 62,3 (**C**H₂-OH) ; 59,9 (C**H₂**-α-N) ; 58,1 (C**H₂**-α-N) ; 57,5 (**C**H₂-α-N × 2) ; 55,5 (**C**H₂-α-N × 2) ; 54,0 (**C**H₂-α-N × 2) ; 51,8 (C**H₂**-α-N) ; 51,3 (**C**H₂-α-N) ; 50,6 (**C**H₂-α-N × 2) ; 33,3 (**C**H₂-γ-N) ; 29,8 (**C**H-β-N) ; 24,6 (**C**H₂-β-N).
**Spectrométrie de masse (MALDI-TOF) :** m/z= 450,34 gmol⁻¹ (M+1)^{+.}
**Analyse élémentaire :** Calculée = C 63,39; H 7.98; N 10,56. Trouvée = C 63,67; H 8,11; N 10,33.

### 2) Cyclam « cross bridge » C-CH₂CH₂OH (10)

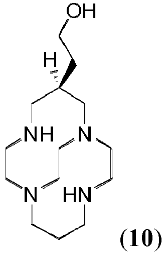

Le mode opératoire est celui utilisé pour la synthèse du composé **(7).** Les réactifs mis en jeu sont : a) composé **(9)** (700 mg ; 1,32 mmol) ; b) Pd/C,10 % (140 mg). Le mode opératoire diverge par l'ajout quotidien de 50 mg de Pd/C au mélange réactionnel. Le composé attendu est une huile jaune **(C₁₄H₃₉N₄O,** M= 270,24 g.mol⁻¹ ; 357 mg ; 1,32 mmol ; rendement 48 %).
**RMN ¹³C (300 MHz, 298K, CDCl₃, δ en ppm) :** 62,7 (**C**H₂-OH) ; 59,4 (C**H₂**-α-N) ; 59,1 (C**H₂**-α-N) ; 58,3 (**C**H₂-α-N × 2) ; 55,3 (**C**H₂-α-N × 2) ; 54,4 (**C**H₂-α-N × 2) ; 52,0 (**C**H₂-α-N) ; 51,3 (**C**H**₂**-α-N) ; 50,6 (**C**H₂-α-N × 2) ; 33,8 (**C**H₂-γ-N) ; 30,0 (**C**H-β-N) ; 24,9 (**C**H₂-β-N).
**Spectrométrie de masse (MALDI-TOF) :** m/z= 271,26 gmol⁻¹ (M+1)⁺

### Exemple 4 : Cyclam-N-monofonctionnalisé et C-fonctionnalisé (13) et Cyclam-amide C-fonctionnalisé (13')

### 1) Préparation du cyclam-amide N-monobenzyle C-CH₂CH₂OH (12)

### Cyclam cis-glyoxal-amide N-monobenzyle C-CH₂CH₂OH (11)

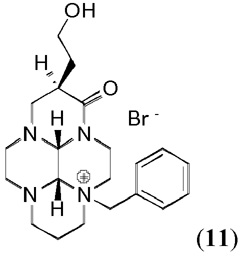

Une solution de bromure de benzyle (1,46 g ; 8,56 mmol) dans 20 mL d'acétonitrile distillé est ajoutée, goutte à goutte, à une solution de cyclam-*cis*-glyoxal-amide C-CH₂CH₂OH **(1)** (2,00 g ; 7,13 mmol) dans 80 mL d'acétonitrile distillé préalablement chauffé à 40 °C.

L'agitation est maintenue pendant 4 jours. Après évaporation du solvant, 25 mL d'eau distillée sont ajoutés au solide jaune obtenu. L'excès de bromure de benzyle est extrait à l'hexane (3 X 30 mL). La phase aqueuse est évaporée pour obtenir le composé attendu sous forme d'un solide jaune (**C₂₁H₃₁N₄BrO₂,** M= 451,16 g.mol⁻¹ ; 3,15 g; 6,98 mmol; rendement 98 %).
**RMN ¹H (300 MHz, 298K, D₂O, δ en ppm)** : 7,64 (d, *J*=7,7 Hz, 2H, H-Ar) ; 7,31 (m, 3H, H-Ar) ; 5,96 (s, 1H, C*H*) ; 5,76 (d, *J*=7,7 Hz, 1H, C*H*₂-Ar) ; 5,59 (d, *J*=7,7 Hz, 1H, C*H*₂-Ar) ; 4,59 (s, 1H, C*H*) ; 4,56 (m, 1H, C*H*₂-α-N) ; 4,33 (m, 1H, O*H*) ; 4,24 (m, 2H, C*H*₂-α-N × 2) ; 3,97 (m, 1H, C*H*₂-α-N) ; 3,77 (m, 2H, C*H*₂-OH) ; 3,59 (m, 1H, C*H*₂-α-N) ; 3,43 (m, 1H, C*H*₂-α-N) ; 3,31 (m, 1H, C*H*₂-α-N) ; 3,18 (m, 1H, C*H*₂-α-N) ; 2,94 (m, 1H, C*H*-β-N) ; 2,87 (m, 2H, C*H*₂-α-N × 2) ; 2,71 (m, 1H, C*H*₂-α-N) ; 2,48 (m, 1H, C*H*₂-α-N) ; 2,08 (m, 2H, C*H*₂-β-N, C*H*₂-γ-N) ; 1,79 (m, 2H, C*H*₂-β-N, C*H*₂-γ-N).
**RMN ¹³C (300 MHz, 298K, D₂O, δ en ppm) :** 177,6 (**C**O), 128,0 (**C**-Ar) ; 136,2 (**C**H-Ar × 2) ; 132,5 (**C**H-Ar × 2) ; 134,3 (**C**H-Ar) ; 83,4 (**C**H) ; 68,0 (**C**H) ; 65,9 (**C**H₂-Ar); 64,3(**C**H₂-α-N) ; 62,6 (**C**H₂-OH) ; [55,8; 55,2; 54,4; 50,0; 45,5; 38,8] (**C**H₂-α-N); 35,8 (***C***H-β-N) ; 34,0 (***C***H₂-γ-N) ; 20,6(***C***H₂-β-N).
**Spectrométrie de masse (MALDI-TOF)** : m/z= 371,26 gmol⁻¹ (M)^{+.}
**IR:** υ_{amide} = 1616 cm⁻¹.
**Analyse élémentaire :** Calculée = C 55,88; H 6,92; N 12,41. Trouvée = C 55,68; H 6,98; N 12,09.

### Cyclam-amide N-monobenzyle C-CH₂CH₂OH (12)

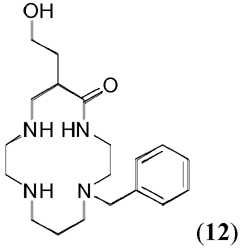

Le mode opératoire est celui utilisé pour la synthèse du composé **5.** Les réactifs mis en jeu sont : a) composé **(11)** (550 mg ; 1,22 mmol) ; b) hydrate d'hydrazine (3 mL). Le produit obtenu se présente sous la forme d'une huile incolore **(C₁₉H₃₂N₄O₂,** M= 348,25 g.mol⁻¹ ; 327 mg ; 0,94 mmol ; rendement 77 %).
**RMN ¹H (300 MHz, 298K, CDCl₃, δ en ppm)** : δ (ppm) 7,26 (m, 5H, **H**-Ar) ; 3,86 (d, J=16 Hz, 1H, C**H**₂-Ar) ; 3,55 (m, 2H, C**H**₂-OH) ; 3,12 (d, J=16 Hz, 1H, C**H**₂-Ph) ; 3,05 (m, 1H, C**H**-β-N) ; 2,20-2,90 (m, 14H, C**H₂**-α-N) ; 2,00 (m, 1H, C**H₂**-β-N) ; 1,73 (m, 1H, C**H**₂-γ-N) ; 1,65 (m, 1H, C*H*₂-y-N) ; 1,55 (m, 1H, C**H**₂-β-N).
**RMN ¹³C (300 MHz, 298K, CDCl₃, δ en ppm)** : 175,7 (CO) ; [138,5 ; 129,5 ; 128,0 ; 126,9] (**C**-Ar, **C**H-Ar) ; 59,7 (_{C}H₂-OH) ; 57,7 (**C**H₂-Ar) ; [54,6 ; 52,8 ; 50,9 ; 50,4 ; 49,3 ; 47,4 ; 42,5] ; 35,8 (**C**H₂-γ-N) ; 33,1 (**C**H-β-N) ; 25,2 (**C**H₂-β-N).
**Spectrométrie de masse (MALDI-TOF) :** m/z= 349,27 gmol⁻¹ (M+1)⁺
**IR:** υ_{amide} = 1616 cm⁻¹.
**Analyse élémentaire :** Calculée = **C** 65,48; H 9.26; N 16,08. Trouvée = **C** 65,69; H 9,44; N 16,11.

### 2) Préparation du cyclam N-monobenzyle C-CH₂CH₂OH (13)

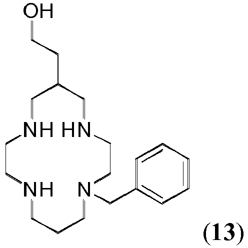

Sous atmosphère d'azote 2,4 ml d'une solution de complexe borane : tétrahydrofurane (BH₃.THF ; 1M ; 2,40 mmol) sont ajoutés à une solution de cyclam-amide-monobenzyle **(12)** (140 mg ; 0,40 mmol) dans 30 mL de THF distillé. Le milieu réactionnel est porté à reflux pendant 2 jours. 30 mL d'eau distillée sont additionnés au mélange, refroidi à 0 °C, pour détruire l'excès de complexe BH₃.THF. Après évaporation du solvant, 25 mL d'une solution d'acide chlorhydrique 3M sont ajoutés au solide blanc obtenu. La solution est portée à reflux pendant 1 heure puis est refroidie à 0 °C. Des pastilles de soude sont additionnées jusqu'à pH = 12. Le produit est extrait au chloroforme (3 x 30 mL) ; les phases organiques recueillies, sont séchées sur du sulfate de magnésium puis évaporées pour donner le composé attendu sous la forme d'une huile incolore (**C₁₉H₃₄N₄O,** M= 334,27 g.mol⁻¹ ; 87 mg ; 0,26 mmol ; rendement 65 %).
**RMN** ¹³**C (300 MHz, 298K, CDCl₃, δ en ppm) :** [138,7 ; 129,4 ; 128,3 ; 127,2](C-Ar, CHAr) ; [59,3 ; 57,9 ; 53,5 ; 52,8 (X 2); 52,6 ; 48,6 ; 48,5 ; 47,8 ; 46,6] (CH₂-OH, CH₂-α-N) ; [35,5 ; 35,3](CH-β-N, CH₂-γ-N) ; 26,2 (CH₂-β-N).
**Spectrométrie de masse (MALDI-TOF)** : m/z= 335,29 gmol⁻¹ (M+1)^{+.}

### 3) Préparation du cyclam-amide N-monobenzyle C-CH₂CH₂OH (13')

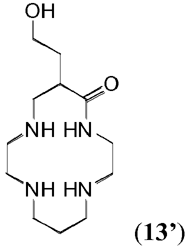

Le mode opératoire est celui utilisé pour la synthèse du composé (7). Les réactifs mis en jeu sont : a) composé (**12**) (700 mg ; 2,01 mmol) ; b) Pd/C, 10 % (140 mg). Le composé attendu est une huile jaune (**C₁₂H₂₆N₄O₂**, M= 258,21 gmol⁻¹ ; 415 mg ; 1,61 mmol ; rendement 80 %).
**RMN ¹³C (300 MHz, 298K, CDCl₃, δ en ppm) :** 176,1 (CO) ; 59,6 (CH₂-OH) ; [51,3 ; 51,1 ; 50,7 ; 50,1 ; 49,2 ; 47,1 ; 42,3] ; 35,5 (CH₂-γ-N) ; 33,9 (CH-β-N) ; 25,7 (CH₂-β-N).
**Spectrométrie de masse (MALDI-TOF)** : m/z= 259,22 gmol⁻¹ (M+1)^{+.}
**IR:** υ_{amide} = 1616 cm⁻¹.

### Exemple 7 : Cyclam-« side bridge » N- et C-fonctionnalisé

### 1) Préparation du cyclam-« side bridge » N-monobenzyle C-CH₂CH₂OH (14)

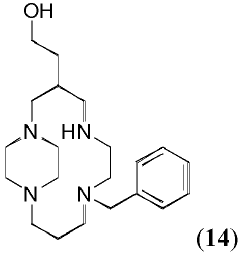

Une solution de (**11**) (205,90 mg ; 0,55 mmol) dans de l'eau distillée est additionnée lentement et à basse température à du borohydrure de sodium (333,00 mg ; 8,80 mmol). Le mélange est agité et porté au reflux durant 5 heures. Après évaporation du solvant, le produit est extrait plusieurs fois avec du chloroforme puis filtré. L'huile incolore obtenue est purifiée par chromatographie sur gel de silice (**C₂₁H₃₆N₄O**, M= 360,54 g.mol⁻¹ ; 140 mg ; 0,31 mmol ; rendement 71 %).
**RMN ¹³C (300 MHz, 298K, CDCl₃, δ en ppm) :** [136,6 ; 125,9 ; 125,1 ; 123,8](C-Ar, CHAr) ; [64,2 ; 60,1 ; 57,6 ; 56,5 ; 53,7 ; 53,1 ; 51,1(X 2, pont) ; 50,4(X 2 pont) ; 49,4 ; 36,4] (CH₂-OH, CH₂-α-N) ; [33,1 ; 31,6](CH-β-N, CH₂-γ-N) ; 21,3 (CH₂-β-N).
**Spectrométrie de masse (MALDI-TOF)** : m/z= 361,62 gmol⁻¹ (M+1)^{+.}

### Exemple 8 : Cyclam-C-fonctionnalisé

### 1) Préparation du cyclam-cis-butanedione-amide C-CH₂COOCH₃ (15)

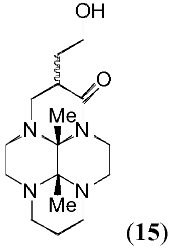

25 ml d'une solution d'α-méthylène-γ-butyrolactone (1,07 g; 9,52 mmol), sont ajoutés, goutte à goutte, à une solution de 2,3,2-butanedione *cis* (2,00 g ; 9,52 mmol) dans 70 mL de méthanol à 45 °C. L'agitation est maintenue pendant 12 jours à 45 °C. Le solvant est évaporé. 15 mL d'une solution de soude 4M sont ajoutés à l'huile marron obtenue. La solution est agitée pendant une nuit. Le produit est extrait au chloroforme (3 X 30 mL). Le composé (**15**) est une huile jaune (**C₁₆H₂₈N₄O₂**, M= 308,22 g.mol⁻¹, 2,05 g ; 6,66 mmol, rendement 70%).
**RMN ¹³C (300 MHz, 298K, CDCl₃, δ en ppm) :** 172,9 (CO) ; 75,5 (CH) ; 72,7 (CH) ; [60,4 ; 50,3 ; 48,8 ; 48,5 ; 46,2 ; 45,6 ; 43,1 ; 38,1] (CH₂-OH, CH₂-α-N) ; [32,9 ; 32,1] (CH-β-N, CH₂-γ-N) ; 24,5 (CH₂-β-N) ; 17,2 (CH₃) ; 10,5 (CH₃).
**Spectrométrie de masse (MALDI-TOF)** : m/z= 309,22 gmol⁻¹ (M+1)^{+.}
**IR :** υ_{amide} = 1616 cm⁻¹.
**Analyse élémentaire** : Calculée = C 62,31; H 9.15; N 18,17. Trouvée = C 62,63; H 9,18; N 18,29.

### 2) Préparation du cyclam C-CH₂CH₂OH (16)

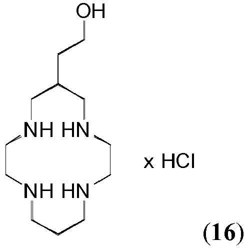

Sous atmosphère d'azote, 7,8 mL d'une solution de complexe borane : tétrahydrofurane (BH₃.THF ; 1M ; 7,80 mmol) sont ajoutés lentement à une solution du composé (**15**) (1,30 mmol, 400 mg) dans 50 mL de tétrahydrofurane distillé. Le mélange est agité pendant 1,5 jours à reflux, sous atmosphère d'azote. Après refroidissement du milieu réactionnel à 0 °C, 30 mL d'eau distillée sont additionnés afin de détruire l'excès de complexe borohydruretétrahydrofurane (BH₃.THF). Après évaporation du mélange, 30 mL d'une solution d'acide chlorhydrique 3M sont ajoutés au solide beige obtenu. La solution est portée à reflux pendant 4 heures. Après évaporation des solvants, le composé (**16**) est obtenu sous forme d'un solide beige (**C₁₂H₅₂N₄OCl₄**, M= 388,13 gmol⁻¹ ; 403 mg ; 1,04 mmol, rendement 80%).
**RMN ¹³C (300 MHz, 298K, D₂O, δ en ppm)** : 64,4 (CH₂-OH) ; 51,9 (CH₂-α-N × 2) ; 44,8 (CH₂-α-N × 2) ; 42,9 (CH₂-α-N × 2) ; 42,3 (CH₂-α-N × 2) ; 35,4 (CH-β-N) ; 31,7 (CH₂-γ-N) ; 30,7 (CH₂-β-N).
**Spectrométrie de masse (MALDI-TOF)** : m/z= 245,24 gmol⁻¹ (M+1)^{+.}

### 3) Préparation du cyclam N-tétra acétate tertiobutyle C-CH₂CH₂OH (17)

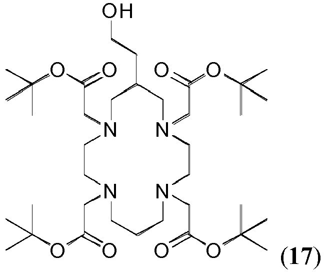

Une solution de bromoacétate de tertiobutyle (796 mg ; 4,08 mmol) dans 45 mL d'acétonitrile distillé est ajoutée, goutte à goutte, à une solution de cyclam C-CH₂CH₂OH (16) (250 mg ; 1,02 mmol) et de carbonate de potassium (845 mg ; 6,12 mmol) dans 80 mL d'acétonitrile distillé, préalablement chauffée à 40°C. La solution est agitée pendant 6 jours à 40 °C puis filtrée sur célite. Après évaporation du solvant, l'huile jaune obtenue est solubilisée dans le chloroforme puis lavée à l'eau (3 x 30 mL). Le produit est purifié par chromatographie sur gel de silice avec comme éluant un mélange CHCl₃/CH₃OH (proportions en v/v ; 100/0 ; 99/1 ; 98/2 ; 97/3 ; 96/4 ; 95/5). Le composé est obtenu sous la forme d'une huile jaune (**C₃₆H₆₈N₄O₉**, M= 700,50 g.mol⁻¹ ; 215mg, 0,31 mmol ; rendement 60 %).
**RMN ¹³C (300 MHz, 298K, CDCl₃, δ en ppm)** : 170,9 (CO ester × 2) ; 170,5 (CO ester × 2) ; 81,2 (C(CH₃)₃ × 2) ; 80,9 (C(CH₃)₃ × 2) ; 61,3 (CH₂-OH) ; 60,5 (CH₂-α-N × 2) ; 57,4 (CH₂-α-N × 2) ; 56,9 (CH₂-α-N × 2) ; 51,8 (CH₂-α-N × 2) ; 51,1 (CH₂-α-N × 2) ; 50,9 (CH₂-α-N × 2) ; 37,7 (CH-β-N) ; 36,0 (CH₂-γ-N) ; 28,3 (C(CH₃)₃ × 12) ; 25,4 (CH₂-β-N).
**Spectrométrie de masse (MALDI-TOF)** : m/z= 701,53 g.mol⁻¹ (M+1)^{+.}

### 4) Préparation du cyclam N-tétra acétate C-CH₂CH₂OH (18)

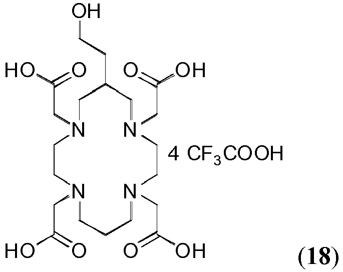

Le composé (**17**) (100 mg ; 0,14 mmol) est dissous dans 10 mL d'acide trifluoroacétique. La solution est agitée pendant 18 heures à température ambiante. Le solvant est évaporé. Le composé (**18**) est obtenu sous la forme d'un solide marron (**C₂₈H₄₀F₁₂N₄O₁₇**, M= 932,62 g.mol⁻¹ ; 130 mg ; 0,14 mmol ; quantitatif).
**RMN ¹³C (300 MHz, 298K**, **D₂O**, **δ en ppm) :** 176,9 (CO acide × 2) ; 178,5 (CO acide × 2) ; 61,3 (CH₂-OH) ; 62,5 (CH₂-α-N × 2) ; 58,4 (CH₂-α-N × 2) ; 57,9 (CH₂-α-N × 2) ; 53,8 (CH₂-α-N × 2) ; 53,1 (CH₂-α-N × 2) ; 51,9 (CH₂-α-N × 2) ; 38,7 (CH-β-N) ; 37,0 (CH₂-γ-N) ; 26,4 (CH₂-β-N).
**Spectrométrie de masse (MALDI-TOF)** : m/z= 476,25 g.mol⁻¹ (M)^{+.} (C₂₀H₃₆N₄O₉)

## Revendications

1. Composé répondant à la formule (I) ou à la formule (II), ses sels ou solvates: 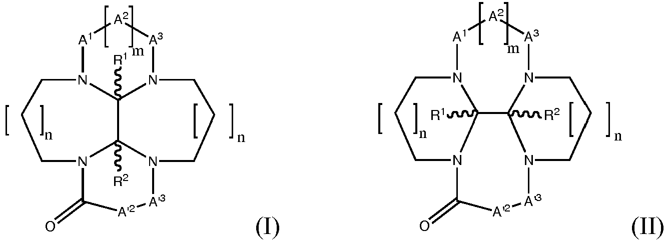 formules (I) et (II) dans lesquelles :
R¹ et R², identiques ou différents, représentent un atome d'hydrogène, un groupe alkyle en C₁-C₄, ou peuvent former ensemble avec les atomes de carbone du pont bisaminal auxquels ils sont reliés un groupe cyclique hydrocarboné à 6 membres, saturé ou insaturé,
m et n, identiques ou différents, sont égaux à zéro ou à un ;
A¹, A², A³, A'², A'³, identiques ou différents, sont choisis parmi les groupes CH₂, CHR et C=O ; sous réserve que A'² ou A'³ désigne un groupe CHR ;
- le ou les groupes R, identiques ou différents, ioniques ou non ioniques, sont choisis parmi :
- un groupe hydrocarboné, saturé ou insaturé, linéaire ou ramifié, cyclique ou acyclique, comprenant de 1 à 30 atomes de carbone, et comprenant éventuellement un ou plusieurs hétéroatomes,
- un groupe V, V étant choisi parmi un halogène, un nitrile, NR⁵R⁶, NO₂, OR⁷, COX, NHCOX, -CONR⁵R⁶, (O)ₛPO(OR⁸)(OR⁹), s étant un entier allant de zéro à un, X étant un halogène ou un groupe OR⁵ ; R⁵, R⁶, R⁷, R⁸ et R⁹ étant choisis parmi un atome d'hydrogène et un groupe alkyle, linéaire ou ramifié, saturé ou insaturé, en C₁-C₃₀,
- un groupe W, W étant un groupe hydrocarboné cyclique, pouvant être mono, bi ou tricyclique, saturé ou insaturé, le ou les cycles comportant de 5 à 6 atomes de carbone, et au moins l'un d'eux pouvant être substitué par un ou plusieurs groupes choisis parmi N-chlorosuccinimide, P(C₆H₅)₂, P(O)(C₆H₅)₂, (CH₂)ₚV, p étant un entier allant de zéro à quatre, et/ou pouvant former avec un ou plusieurs atomes de carbone du cycle une ou plusieurs fonctions cétone,
- un groupe Y, Y étant un groupe hétérocyclique, pouvant être mono, bi ou tricyclique, saturé ou insaturé, le ou les cycles comportant de 5 à 6 membres, et au moins l'un d'eux pouvant être substitué sur un ou plusieurs atomes de carbone ou hétéroatome par un ou plusieurs groupes choisis parmi les groupes V et W, un groupe alkyle linéaire ou ramifié, saturé ou insaturé, en C₁-C₃₀, substitué par un ou plusieurs groupes V et/ou W, un groupe trityle ; ledit groupe hétérocycle pouvant former avec un ou plusieurs atomes de carbone du cycle une ou plusieurs fonctions cétone,
- un groupe alkyle en C₁-C₃₀, linéaire ou ramifié, saturé ou insaturé, substitué par un ou plusieurs groupes V, W, et/ou Y ,
- des groupes hydrophobes, choisi parmi les groupes hydrocarbonés, saturés ou insaturés, linéaires ou ramifiés, cycliques ou acycliques, comprenant au moins une chaîne hydrocarbonée linéaire ayant de 8 à 30 atomes de carbone,
- des groupes hydrophiles comportant au moins un groupe éthylène glycol,
- des groupes de type lipide/phospholipide,
- des groupes fluorescents,
- des groupes ioniques choisis parmi des groupes organiques de type guadinidium ou arsenium,
- des groupes porteurs d'au moins une fonction amine primaire, secondaire ou tertiaire éventuellement protégée,
- des groupes porteurs d'au moins une fonction alcool,
- des groupes porteurs d'au moins une fonction alcène et/ou alcyne,
- des groupes porteurs d'au moins une fonction amide,
- des groupes porteurs d'au moins une fonction cyano, nitrile, CF₃,
- des groupes porteurs d'au moins une fonction acide, ester, aldéhyde.

2. Composé selon la revendication 1, **caractérisé en ce que** A'² désigne un groupe CHR, R étant tel que défini dans la revendication 1.

3. Composé selon l'une quelconque des revendications précédentes, **caractérisé en ce qu'**il répond à la formule suivante (III), un de ses sels ou solvates : 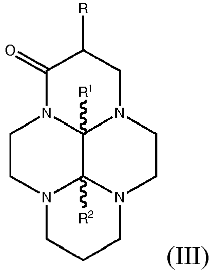 dans laquelle : R¹ et R² sont tels que définis dans la revendication 1 ; R est un groupe (CH₂)ᵢX avec i un entier égal à zéro ou un, X étant un groupe choisi parmi un atome d'halogène, un hydroxyle, CH₂OH, COOH, COOCH₃, COOC₂H₅.

4. Procédé de préparation d'un composé tel que défini dans l'une quelconque des revendications 1 à 3, **caractérisé en ce qu'**il comprend :
(i) une étape au cours de laquelle on fait réagir une tétramine de formule (VII) 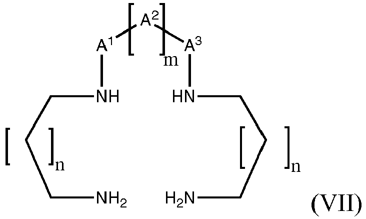
dans laquelle : A¹, A², A³ sont choisis parmi les groupes CH₂, CHR et C=O,
m et n, identiques ou différents, sont égaux à zéro ou un,
avec un composé dicarbonyle de formule (IV) : 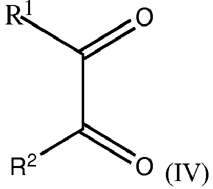 dans laquelle : R¹ et R², identiques ou différents, représentent un atome d'hydrogène, un groupe alkyle en C₁-C₄, ou peuvent former ensemble avec les atomes de carbone auxquels ils sont reliés un groupe cyclique hydrocarboné à 6 atomes de carbone, saturé ou insaturé,
pour former un intermédiaire bisaminal de formule (VIII), (IX), ou un mélange de ces composés : 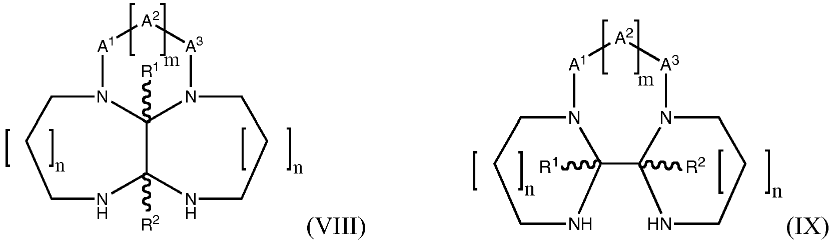 formules (VIII) et (IX) dans lesquelles : A¹, A², A³, m, n sont tels que définis dans la formule (VII) et R¹ et R² sont tels que définis dans la formule (IV) ;
(ii) puis une étape au cours de laquelle on fait réagir le ou les intermédiaires obtenus avec un agent cyclisant répondant à la formule (VI) : 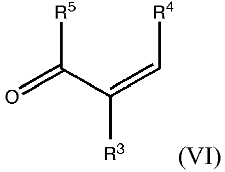 dans laquelle :
R³ et R⁴, identiques ou différents, sont chacun un groupe R tel que défini dans la formule (I) ou (II) définie dans l'une quelconque des revendications 1 à 3;
R⁵ représente un hydroxyle, un halogène, un alcoxyle ; ou
R⁵ peut former avec R³ ou R⁴ un groupe cyclique.

5. Procédé de préparation d'un composé tel que défini dans l'une quelconque des revendications 1 à 3, **caractérisé en ce qu'**il comprend :
(i) une étape au cours de laquelle on fait réagir au moins deux équivalents d'éthylène diamine ou de propylène diamine avec au moins un équivalent d'un composé dicarbonyle de formule (IV) tel que défini dans la revendication précédente,
pour former un intermédiaire bisaminal de formule (V) ou (V') ou un mélange de ces composés: 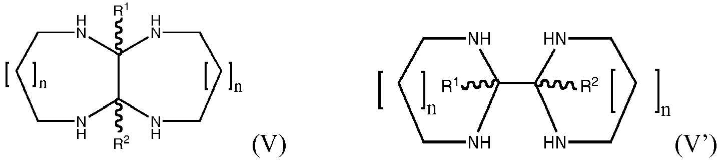 formule (V) et (V') dans lesquelles R¹ et R² sont tels que définis dans la formule (IV) définie à la revendication précédente;
(ii) puis une étape au cours de laquelle on fait réagir l'intermédiaire obtenu avec un premier agent cyclisant, répondant à la formule (VI) telle que défini dans la revendication précédente ;
(iii) et une étape au cours de laquelle on fait réagir le nouvel intermédiaire obtenu avec un deuxième agent cyclisant répondant à la formule (VI) telle que défini dans la revendication précédente ou constitué par un composé Q-CH₂CH₂CH₂-Q' avec Q et Q' choisi parmi des groupes partants tels que des halogènes ou un groupe tosylate
les étapes (ii) et (iii) pouvant être effectuées simultanément ou successivement.

6. Procédé selon la revendication 4 ou 5, **caractérisé en ce que** l'agent cyclisant de formule (VI) est tel que R⁵ forme avec R³ ou R⁴ un groupe cyclique répondant à l'une des formules (X) ou (XI) : 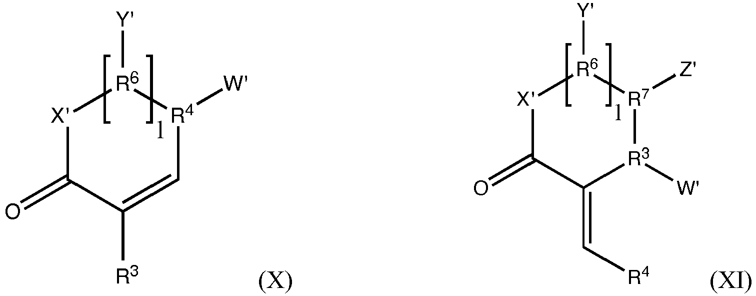
formule (X) dans laquelle : R³ est un groupe R tel que défini dans la formule (I) ou (II) définie dans l'une quelconque des revendications 1 à 3,
X' est un atome d'oxygène,
Y' et W', identiques ou différents, sont choisis parmi un atome d'hydrogène, un groupe alkyle, linéaire ou ramifié, saturé ou insaturé, en C₁-C₃₀,
1 est égal à zéro ou un, avec
- lorsque 1 est égal à zéro, le groupe X' est relié au groupe R⁴ et R⁴ formant avec W' un groupe CH₂ ou C=O,
- lorsque 1 est égal à un, R⁴ et R⁶ forment ensemble avec les groupes Y' et W' des groupes CH₂-CH₂, CH=CH, CH₂(C=O), (C=O)CH₂ ou un groupe phényle ;
formule (XI) dans laquelle : R⁴ est un groupe R, tel que défini dans la formule (I) ou (II) définie dans l'une quelconque des revendications 1 à 3,
X' est un atome d'oxygène
W', Y' et Z', identiques ou différents, sont choisis parmi un atome d'hydrogène, un groupe alkyle, linéaire ou ramifié, saturé ou insaturé, en C₁-C₃₀,
R³, R⁶ et R⁷ désignant des groupes CH₂ et
1 est égal à zéro ou un, avec
- lorsque 1 est égal à un,
R³ et R⁷ pouvant former ensemble avec les groupes Z' et W' les groupes : CH₂-CH₂, CH=CH, ou phényle, et/ou
R⁶ et R⁷ pouvant former ensemble avec les groupes Y' et Z' les groupes : CH₂-CH₂, CH=CH, ou phényle
- lorsque 1 est égal à zéro, le groupe X' étant relié au groupe R⁷.

7. Procédé selon l'une quelconque des revendication 4 à 6, **caractérisé en ce que** les étapes (i), (ii) et/ou (iii) ont lieu dans un milieu protique et/ou sont réalisées sans étapes intermédiaires de séparation ou de purification.

8. Procédé de préparation d'un composé cyclique tétraazoté, dérivé du cyclam, C-fonctionnalisé et/ou N-fonctionnalisé, à partir d'un composé de formule (I), (II), un de ses sels, solvates ou leur mélange, tel que défini dans l'une quelconque des revendications 1 à 3, comprenant au moins l'une des étapes suivantes :
A) substitution d'un ou plusieurs des quatre atomes d'azote du composé (I) et/ou (II) par un ou plusieurs groupes R", identiques ou différents, R" pouvant désigner un groupe fonctionnel R, ou précurseur de groupe fonctionnel R, tel que défini dans l'une des revendications 1 à 3, ou groupe protecteur de fonction amine,
B) réduction d'une ou plusieurs fonctions cétone en position alpha d'un des quatre atomes d'azote du composé (I) et/ou (II), le(s)dit(s) composé(s) (I) et/ou (II) étant éventuellement modifié(s) par une ou plusieurs étapes A) et/ou C),
C) déprotection du pont bisaminal du composé (I) et/ou (II), le(s)dit(s) composé(s) (I) et/ou (II) étant éventuellement été modifié(s) par une ou plusieurs étapes A) et/ou B), les étapes B) et C) pouvant être réalisées simultanément ou séparément ;
ledit composé cyclique tétraazoté, dérivé du cyclam, C-fonctionnalisé et/ou N-fonctionnalisé étant de formule choisie parmi le groupe: 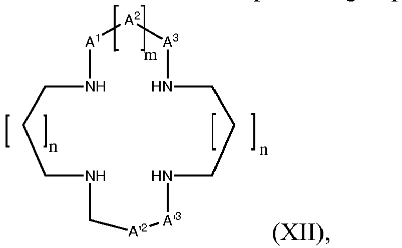 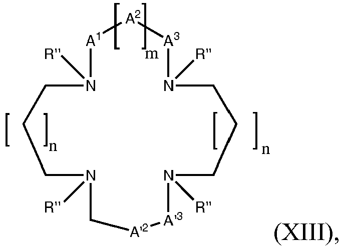 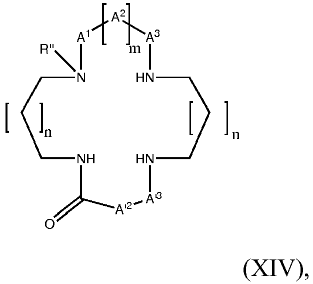 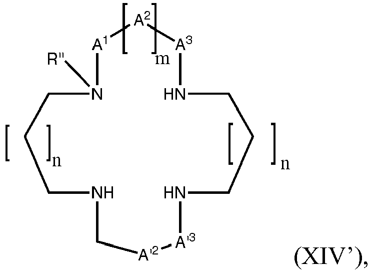 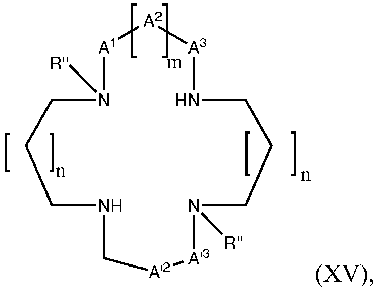 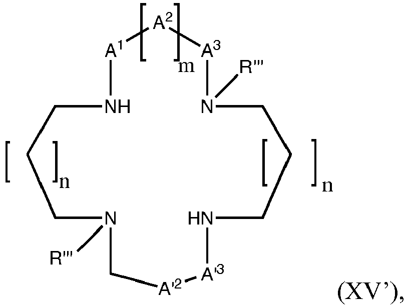 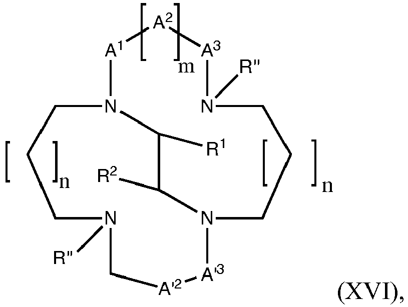 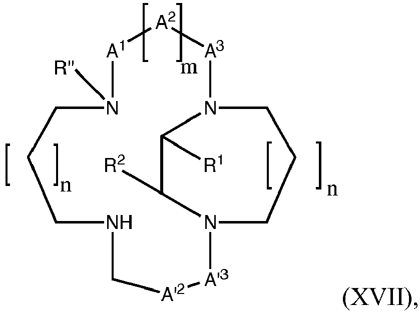 et 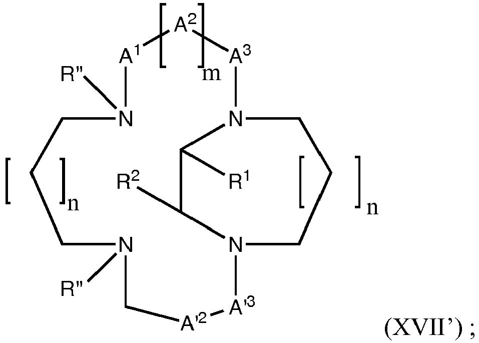 dans laquelle : A¹, A², A³, A'², A'³, R¹ et R², m et n, sont tels que définis dans la revendication 1 ou 2,
les groupes R", identiques ou différents, désignant un groupe fonctionnel R, ou précurseur de groupe fonctionnel R, tel que défini dans l'une des revendications 1 à 3, ou un groupe protecteur d'un atome d'azote du composé cyclique tétraazoté.
les groupes R'", identiques ou différents, de préférence identiques, correspondent à des groupes fonctionnels R", ou précurseur de groupe fonctionnel R".

9. Procédé de préparation, selon la revendication 8, d'un composé de formule (XII) suivante, un de ses sels, solvates ou mélange: 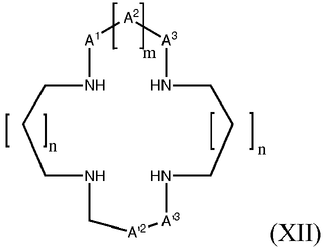 dans laquelle : A¹, A², A³, A'², A'³, m et n, sont tels que définis dans la revendication 1 ou 3, ledit procédé comprenant une étape de réduction du composé de formule (I) et/ou (II) tel que défini dans l'une quelconque des revendications 1 ou 2.

10. Procédé de préparation, selon la revendication 8, d'un composé de formule (XIII) suivante, un de ses sels, solvates ou mélange: 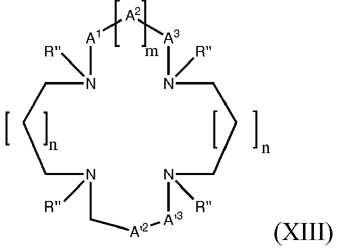 dans laquelle : A¹, A², A³, A'², A'³, m et n sont tels que définis dans la revendication 1 ou 2, les groupes R", identiques ou différents, désignant un groupe fonctionnel R, ou précurseur de groupe fonctionnel R, tel que défini dans l'une des revendications 1 à 3, ou un groupe protecteur d'un atome d'azote du composé cyclique tétraazoté, ledit procédé comprenant les étapes suivantes :
(i) réduction d'un composé de formule (I) et/ou (II), tel que défini précédemment, et enfin
(ii) substitution, simultanée ou successive, sur les quatre atome d'azote du cycle tétraazoté du composé de formule (I) et/ou (II) réduit , par des groupes R", identiques ou différents.

11. Procédé de préparation, selon la revendication 8, d'un composé C-fonctionnalisé et N-mono-fonctionnalisé, un de ses sels, solvates, ou mélange, comprenant les étapes suivantes :
(i) substitution d'un atome d'azote d'un composé de formule (I) et/ou (II), par un groupe protecteur R" tel que défini dans la formule (XIII) dans la revendication 10, puis
(ii) déprotection du pont bisaminal avec de l'hydrate d'hydrazine ou de la soude, pour obtenir le composé de formule (XIV) suivante, un de ses sels, solvates, ou mélange : 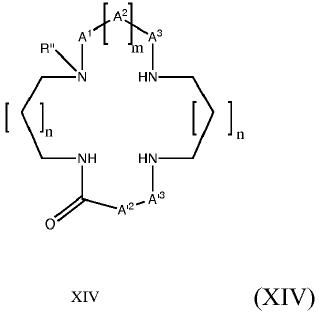 dans laquelle : A¹, A², A³, A'², A'³, m et n sont tels que définis dans la revendication 1 ou 2, le groupe R" est tel que défini dans la formule (XIII) dans la revendication 10,
(iii) éventuellement réduction du composé de formule (XIV).

12. Procédé de préparation, selon la revendication 8, d'un composé C-fonctionnalisé et N--fonctionnalisé, un de ses sels, solvates, ou mélange, comprenant les étapes suivantes :
(i) réduction d'un composé cyclique de formule (I) et/ou (II), tel que défini dans l'une des revendications 1 à 3, ou de leur mélange, à l'aide d'un agent réducteur sélectif, puis
(ii) substitution sur deux atomes d'azote non adjacent du cycle tétraazoté de formule (I) ou, (II) réduit, ou (I) et (II) réduit en cas de mélange, ou (III) par des groupes R", et
(iii) déprotection du pont bisaminal avec de l'hydrate d'hydrazine ou de la soude, pour obtenir un composé de formule (XV) suivante, un de ses sels, solvates, ou mélange: 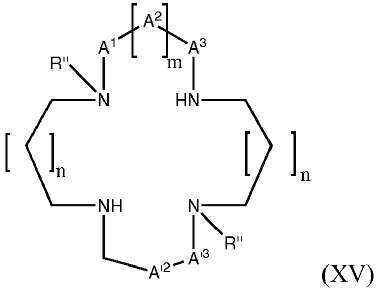 dans laquelle : A¹, A², A³, A'², A'³, m et n sont tels que définis dans la revendication 1 ou 2; les groupes R", identiques ou différents, sont tels que définis dans la formule (XIII) dans la revendication 11,
(iv) et éventuellement lorsque R" est un groupe protecteur de fonction aminé :
- substitution des deux amines secondaires non substituées du composé (XV) par des groupes R"', identiques ou différents, désignant un groupe fonctionnel R", ou précurseur de groupe fonctionnel R", tel que défini dans la revendication 11, puis
- déprotection des groupes protecteurs R".

13. Procédé de préparation, selon la revendication 8, d'un composé de formule (XVI) suivante, un de ses sels, solvates, ou mélange: 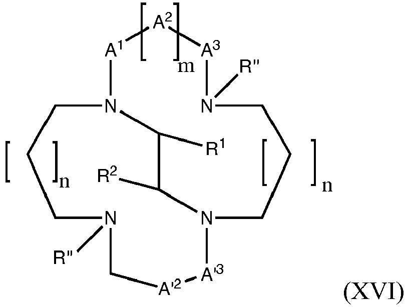 dans laquelle : A¹, A², A³, A'², A'³, R¹ et R², m et n sont tels que définis dans la revendication 1 ou 2, les groupes R", identiques ou différents, sont tels que définis dans la formule (XIII) dans la revendication 11, ledit procédé comprenant les étapes suivantes:
(i) réduction de la fonction cétone d'un composé de formule (I) et/ou (II) tel que défini dans l'une des revendications 1 à 3, à l'aide d'un agent réducteur sélectif, puis
(ii) substitution des amines secondaires du composé de formule (I) et/ou (II) réduit et enfin
(iii) réduction partielle du pont bisaminal à l'aide d'un agent réducteur sélectif.

14. Procédé de préparation, selon la revendication 8, d'un composé C-fonctionnalisé et N-(mono et évenutellement di)-fonctionnalisé, un de ses sels, solvates, ou mélange, comprenant les étapes suivantes:
(i) substitution d'une amine secondaire du composé (I) et/ou (II), par un groupe R", tel que défini dans la formule (XIII) dans la revendication 11, puis
(ii) réduction partielle du pont bisaminal à l'aide d'un agent réducteur sélectif, pour obtenir un composé de formule (XVII), un de ses sels, solvates, ou mélange: 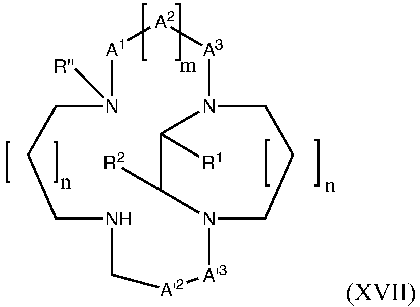 dans laquelle : A¹, A², A³, A'², A'³, R¹ et R², m et n sont tels que définis dans la revendication 1 ou 2, le groupe R" est tel que défini dans la formule (XIII) dans la revendication 11,
et éventuellement : (iii) substitution de l'amine secondaire du composé (XVII) par un groupe R" tel que défini dans la formule (XIII) défini dans la revendication 11, pour obtenir un composé de formule (XVII'), un de ses sels, solvates, ou mélange: 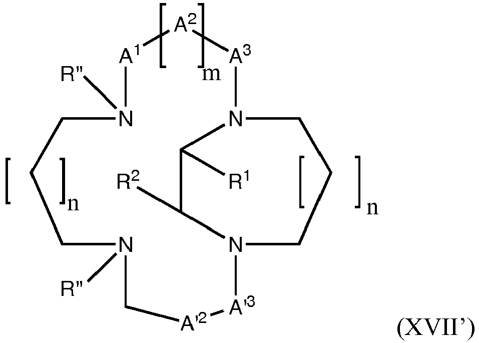 dans laquelle : A¹, A², A³, A'², A'³, R¹ et R², m et n sont tels que définis dans la formule (I) ou (II) défini dans l'une quelconque des revendications 1 à 3, les groupes R", identiques ou différents, sont tels que définis dans la formule (XIII) défini dans la revendication 11.

## Patentansprüche

1. Verbindung der Formel (I) oder der Formel (II), Salze oder Solvate davon: 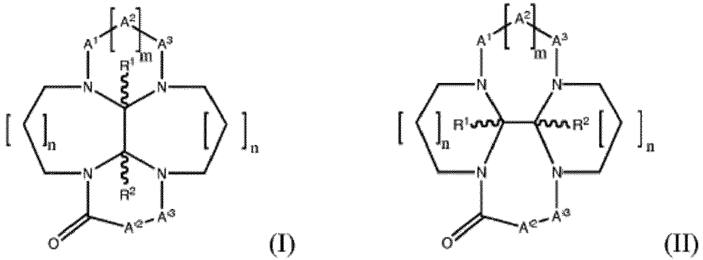 wobei in den Formeln (I) und (II):
R¹ und R², die gleich oder verschieden sind, für ein Wasserstoffatom oder eine C₁-C₄-Alkylgruppe stehen oder zusammen mit den Kohlenstoffatomen der Bisaminal-Brücke, an die sie gebunden sind, eine gesättigte oder ungesättigte 6-gliedrige cyclische Kohlenwasserstoffgruppe bilden können,
m und n, die gleich oder verschieden sind, gleich null oder eins sind;
A¹, A², A³, A'² und A'³, die gleich oder verschieden sind, aus den Gruppen CH₂, CHR und C=O ausgewählt sind; mit der Maßgabe, dass A'² oder A'³ für eine CHR-Gruppe stehen;
- die Gruppe bzw. die Gruppen R, die gleich oder verschieden und ionisch oder nichtionisch sind, aus:
- einer cyclischen oder acyclischen, linearen oder verzweigten, gesättigten oder ungesättigten Kohlenwasserstoffgruppe mit 1 bis 30 Kohlenstoffatomen und gegebenenfalls einem oder mehreren Heteroatomen,
- einer Gruppe V, wobei V aus einem Halogen, einem Nitril, NR⁵R⁶, NO₂, OR⁷, COX, NHCOX, -CONR⁵R⁶ und (O)ₛPO(OR⁸)(OR⁹) ausgewählt ist, wobei s eine ganze Zahl im Bereich von null bis eins ist, X ein Halogen oder eine Gruppe OR⁵ ist und R⁵, R⁶, R⁷, R⁸ und R⁹ aus einem Wasserstoffatom und einer gesättigten oder ungesättigten, linearen oder verzweigten C₁-C₃₀-Alkylgruppe ausgewählt sind,
- einer Gruppe W, wobei W eine gesättigte oder ungesättigte cyclische Kohlenwasserstoffgruppe ist, die mono-, bi- oder tricyclisch sein kann, wobei der Ring bzw. die Ringe 5 bis 6 Kohlenstoffatome enthält bzw. enthalten und mindestens einer davon durch eine oder mehrere Gruppen, die aus N-Chlorsuccinimid, P(C₆H₅)₂, P(O)(C₆H₅)₂ und (CH₂)ₚV ausgewählt sind, substituiert sein kann, wobei p eine ganze Zahl im Bereich von null bis vier ist, und/oder mit einem oder mehreren Kohlenstoffatomen des Rings ein oder mehrere Ketonfunktionen bilden kann,
- einer Gruppe Y, wobei Y eine gesättigte oder ungesättigte heterocyclische Gruppe ist, die mono-, bi- oder tricyclisch sein kann, wobei der Ring bzw. die Ringe 5- oder 6-gliedrig ist bzw. sind und mindestens einer davon an einem oder mehreren Kohlenstoff- oder Heteroatomen durch eine oder mehrere Gruppen, die aus den Gruppen V und W, einer gesättigten oder ungesättigten, linearen oder verzweigten C₁-C₃₀-Alkylgruppe, die durch eine oder mehrere Gruppen V und/oder W substituiert ist, und einer Tritylgruppe ausgewählt sind, substituiert sein kann; wobei die Heterocyclusgruppe mit einem oder mehreren Kohlenstoffatomen des Rings ein oder mehrere Ketonfunktionen bilden kann,
- einer gesättigten oder ungesättigten, linearen oder verzweigten C₁-C₃₀-Alkylgruppe, die durch eine oder mehrere Gruppen V, W und/oder Y substituiert ist,
- hydrophoben Gruppen, die aus cyclischen oder acyclischen, linearen oder verzweigten, gesättigten oder ungesättigten Kohlenwasserstoffgruppen mit 8 bis 30 Kohlenstoffatomen ausgewählt sind,
- hydrophilen Gruppen mit mindestens einer Ethylenglykolgruppe,
- Gruppen vom Lipid/Phospholipid-Typ,
- fluoreszierenden Gruppen,
- ionischen Gruppen, die aus organischen Gruppen vom Guanidium- oder Arsenium-Typ ausgewählt sind,
- Gruppen mit mindestens einer gegebenenfalls geschützten primären, sekundären oder tertiären Aminfunktion,
- Gruppen mit mindestens einer Alkoholfunktion,
- Gruppen mit mindestens einer Alken- und/oder Alkinfunktion,
- Gruppen mit mindestens einer Amidfunktion,
- Gruppen mit mindestens einer Cyano-, Nitril-, CF₃-Funktion,
- Gruppen mit mindestens einer Säure-, Ester-, Aldehydfunktion
ausgewählt ist bzw. sind.

2. Verbindung nach Anspruch 1, **dadurch gekennzeichnet, dass** A'² für eine CHR-Gruppe steht, wobei R wie in Anspruch 1 definiert ist.

3. Verbindung nach einem der vorhergehenden Ansprüche, **dadurch gekennzeichnet, dass** sie der folgenden Formel (III), einem Salz oder Solvat davon entspricht: 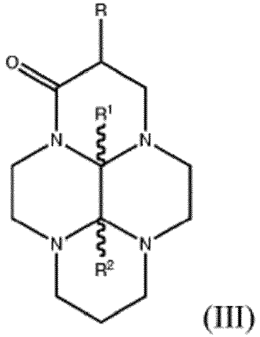 in der: R¹ und R² wie in Anspruch 1 definiert sind; R eine (CH₂)ᵢX-Gruppe ist, wobei i eine ganze Zahl mit einem Wert von null oder 1 ist und X eine Gruppe ist, die aus einem Halogenatom, einem Hydroxyl, CH₂OH, COOH, COOCH₃ und COOC₂H₅ ausgewählt ist.

4. Verfahren zur Herstellung einer Verbindung gemäß einem der Ansprüche 1 bis 3, **dadurch gekennzeichnet, dass** es Folgendes umfasst:
(i) einen Schritt, bei dem man ein Tetramin der Formel (VII) 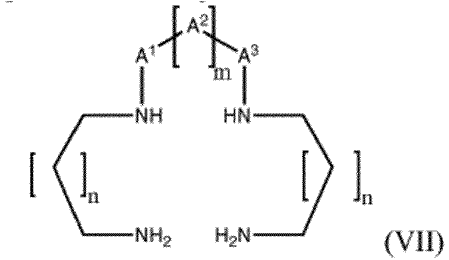 in der: A¹, A² und A³ aus den Gruppen CH₂, CHR und C=O ausgewählt sind, m und n, die gleich oder verschieden sind, gleich null oder eins sind,
mit einer Dicarbonylverbindung der Formel (IV): 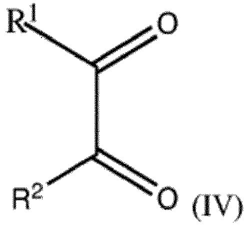 in der: R¹ und R², die gleich oder verschieden sind, für ein Wasserstoffatom oder eine C₁-C₄-Alkylgruppe stehen oder zusammen mit den Kohlenstoffatomen, an die sie gebunden sind, eine gesättigte oder ungesättigte 6-gliedrige cyclische Kohlenwasserstoffgruppe bilden können,
zu einem Bisaminal-Zwischenprodukt der Formel (VIII), (IX) oder einem Gemisch dieser Verbindungen umsetzt: 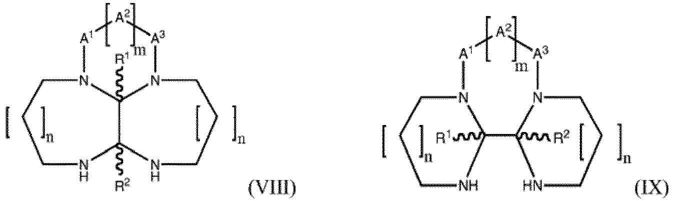 wobei in den Formeln (VIII) und (IX) : A¹, A², A³, m und n wie in der Formel (VII) definiert sind und R¹ und R² wie in der Formel (IV) definiert sind;
(ii) dann einen Schritt, bei dem man das erhaltene Zwischenprodukt bzw. die erhaltenen Zwischenprodukte mit einem Cyclisierungsmittel der Formel (VI) : 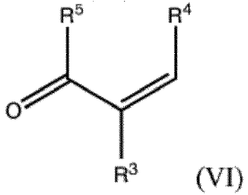 in der:
R³ und R⁴, die gleich oder verschieden sind, jeweils eine Gruppe R gemäß der in der in einem der Ansprüche 1 bis 3 definierten Formel (I) oder (II) angegebenen Definition sind;
R⁵ für ein Hydroxyl, ein Halogen oder ein Alkoxyl steht oder
R⁵ mit R³ oder R⁴ eine cyclische Gruppe bilden kann;
umsetzt.

5. Verfahren zur Herstellung einer Verbindung gemäß einem der Ansprüche 1 bis 3, **dadurch gekennzeichnet, dass** es Folgendes umfasst:
(i) einen Schritt, bei dem man mindestens zwei Äquivalente Ethylendiamin oder Propylendiamin mit mindestens einem Äquivalent einer Dicarbonylverbindung der Formel (IV) gemäß der in dem vorhergehenden Anspruch angegebenen Definition zu einem Bisaminal-Zwischenprodukt der Formel (V) oder (V') oder einem Gemisch dieser Verbindungen umsetzt: 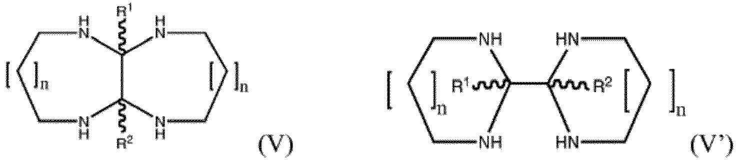 wobei in den Formeln (V) und (V') R¹ und R² wie in der im vorhergehenden Anspruch definierten Formel (IV) definiert sind;
(ii) dann einen Schritt, bei dem man das erhaltene Zwischenprodukt mit einem ersten Cyclisierungsmittel, das der Formel (VI) gemäß der im vorhergehenden Anspruch angegebenen Definition entspricht, umsetzt;
(iii) und einen Schritt, bei dem man das erhaltene neue Zwischenprodukt mit einem zweiten Cyclisierungsmittel, das der Formel (VI) gemäß der im vorhergehenden Anspruch angegebenen Definition entspricht oder aus einer Verbindung Q-CH₂CH₂CH₂-Q' besteht, wobei Q und Q' aus Abgangsgruppen wie Halogenen oder einer Tosylatgruppe ausgewählt sind, umsetzt;
wobei die Schritte (ii) und (iii) gleichzeitig oder nacheinander durchgeführt werden können.

6. Verfahren nach Anspruch 4 oder 5, **dadurch gekennzeichnet, dass** das Cyclisierungsmittel der Formel (VI) so beschaffen ist, dass R⁵ mit R³ oder R⁴ eine cyclische Gruppe einer der Formeln (X) oder (XI) bildet: 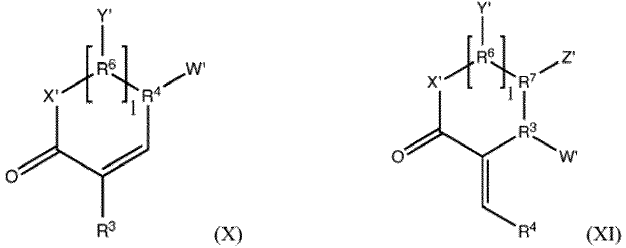
wobei in der Formel (X): R³ eine Gruppe R gemäß der in der in einem der Ansprüche 1 bis 3 definierten Formel (I) oder (II) angegebenen Definition ist,
X' ein Sauerstoffatom ist,
Y' und W', die gleich oder verschieden sind, aus einem Wasserstoffatom und einer gesättigten oder ungesättigten, linearen oder verzweigten C₁-C₃₀-Alkylgruppe ausgewählt sind,
l gleich null oder 1 ist, wobei
- dann, wenn l gleich null ist, die Gruppe X' an die Gruppe R⁴ gebunden ist und R⁴ mit W' eine CH₂-oder C=O-Gruppe bildet;
- dann, wenn l gleich eins ist, R⁴ und R⁶ zusammen mit den Gruppen Y' und W' CH₂-CH₂-, CH=CH-, CH₂(C=O)-, (C=O)CH₂-Gruppen oder eine Phenylgruppe bilden;
wobei in der Formel (XI): R⁴ eine Gruppe R gemäß der in der in einem der Ansprüche 1 bis 3 definierten Formel (I) oder (II) angegebenen Definition ist,
X' ein Sauerstoffatom ist,
W', Y' und Z', die gleich oder verschieden sind, aus einem Wasserstoffatom und einer gesättigten oder ungesättigten, linearen oder verzweigten C₁-C₃₀-Alkylgruppe ausgewählt sind,
R³, R⁶ und R⁷ für CH₂-Gruppen stehen und
l gleich null oder 1 ist, wobei
- dann, wenn l gleich eins ist,
R³ und R⁷ zusammen mit den Gruppen Z' und W' die Gruppen: CH₂-CH₂, CH=CH oder Phenyl bilden können und/oder
R⁶ und R⁷ zusammen mit den Gruppen Y' und Z' die Gruppen: CH₂-CH₂, CH=CH oder Phenyl bilden können,
- dann, wenn l gleich null ist, die Gruppe X' an die Gruppe R⁷ gebunden ist.

7. Verfahren nach einem der Ansprüche 4 bis 6, **dadurch gekennzeichnet, dass** die Schritte (i), (ii) und/oder (iii) in einem protischen Medium stattfinden und/oder ohne zwischengeschaltete Trenn- oder Reinigungsschritte durchgeführt werden.

8. Verfahren zur Herstellung einer C-funktionalisierten und/oder N-funktionalisierten cyclischen Tetrastickstoffverbindung, die sich von Cyclam ableitet, aus einer Verbindung der Formel (I), (II), einem Salz oder Solvat davon oder einem Gemisch davon gemäß einem der Ansprüche 1 bis 3, das mindestens einen der folgenden Schritte umfasst:
A) Substitution eines oder mehrerer der vier Stickstoffatome der Verbindung (I) und/oder (II) durch eine oder mehrere Gruppen R", die gleich oder verschieden sind, wobei R" für eine funktionelle Gruppe R oder einen Vorläufer einer funktionellen Gruppe R gemäß der in einem der Ansprüche 1 bis 3 angegebenen Definition oder eine Schutzgruppe für die Aminfunktion stehen kann,
B) Reduktion einer oder mehrerer Ketonfunktionen in alpha-Position zu einem der vier Stickstoffatome der Verbindung (I) und/oder (II), wobei die Verbindung bzw. Verbindungen (I) und/oder (II) gegebenenfalls durch einen oder mehrere Schritte A) und/oder C) modifiziert wird bzw. werden,
C) Entschützung der Bisaminalbrücke der Verbindung (I) und/oder (II), wobei die Verbindung bzw. Verbindungen (I) und/oder (II) gegebenenfalls durch einen oder mehrere Schritte A) und/oder B) modifiziert wird bzw. werden,
wobei die Schritte B) und C) gleichzeitig oder getrennt durchgeführt werden;
wobei die C-funktionalisierte und/oder N-funktionalisierte cyclische Tetrastickstoffverbindung, die sich von Cyclam ableitet, eine Formel aufweist, die aus der folgenden Gruppe ausgewählt ist: 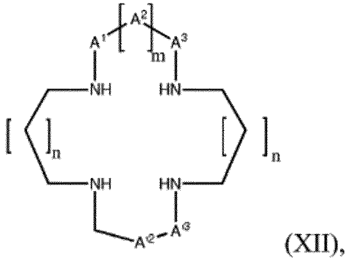 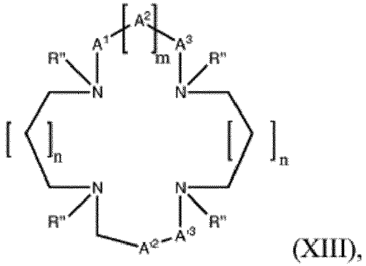 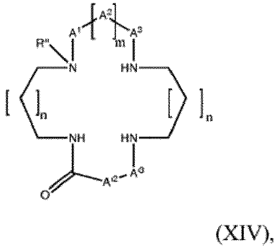 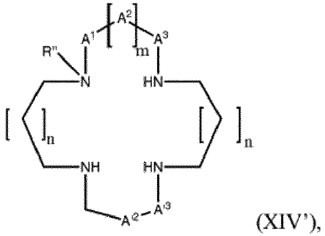 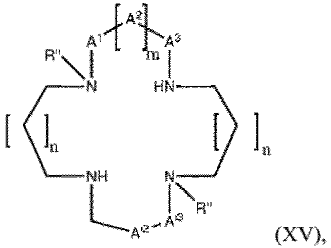 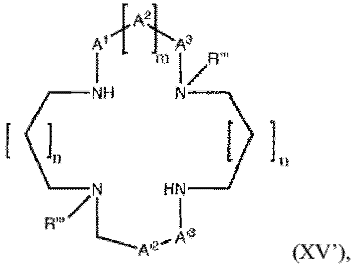 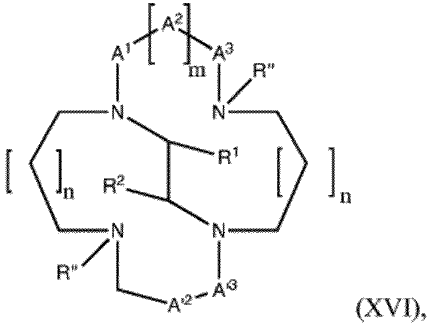 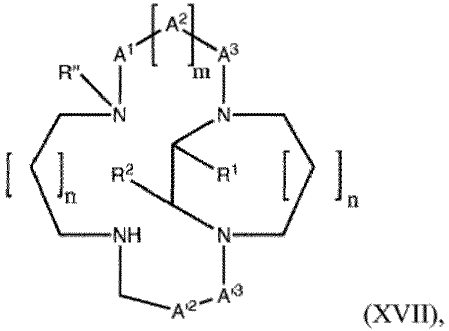 und 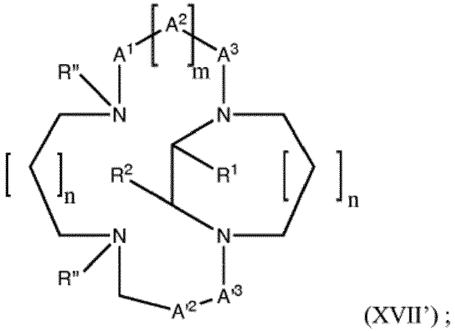 in der: A¹, A², A³, A'², A'³, R¹ und R², m und n wie in Anspruch 1 oder 2 definiert sind,
wobei die Gruppen R", die gleich oder verschieden sind, für eine funktionelle Gruppe R oder einen Vorläufer einer funktionellen Gruppe R gemäß der in einem der Ansprüche 1 bis 3 angegebenen Definition oder eine Schutzgruppe für ein Stickstoffatom der cyclischen Tetrastickstoffverbindung stehen,
wobei die Gruppen R"', die gleich oder verschieden, vorzugsweise gleich, sind, den funktionellen Gruppen R" oder einem Vorläufer einer funktionellen Gruppe R" entsprechen.

9. Herstellungsverfahren nach Anspruch 8 zur Herstellung einer Verbindung der folgenden Formel (XII), eines Salzes, Solvats oder Gemischs davon: 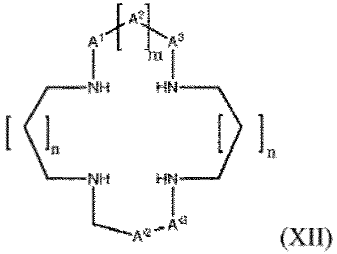 in der: A¹, A², A³, A'², A'³, m und n wie in Anspruch 1 oder 3 definiert sind, wobei das Verfahren einen Schritt der Reduktion der Verbindung der Formel (I) und/oder (II) gemäß der in einem der Ansprüche 1 oder 2 angegebenen Definition umfasst.

10. Herstellungsverfahren nach Anspruch 8 zur Herstellung einer Verbindung der folgenden Formel (XIII), eines Salzes, Solvats oder Gemischs davon: 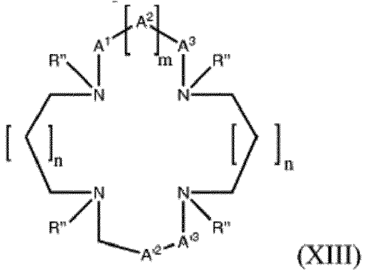 in der: A¹, A², A³, A'², A'³, m und n wie in Anspruch 1 oder 2 definiert sind, wobei die Gruppen R", die gleich oder verschieden sind, für eine funktionelle Gruppe R oder einen Vorläufer einer funktionellen Gruppe R gemäß der in einem der Ansprüche 1 bis 3 angegebenen Definition oder eine Schutzgruppe für ein Stickstoffatom der cyclischen Tetrastickstoffverbindung stehen, wobei das Verfahren folgende Schritte umfasst:
(i) Reduktion einer Verbindung der Formel (I) und/oder (II) gemäß obiger Definition und schließlich
(ii) gleichzeitige oder nachfolgende Substitution an den vier Stickstoffatomen des Tetrastickstoffrings der reduzierten Verbindung der Formel (I) und/oder (II) durch gleiche oder verschiedene Gruppen R".

11. Herstellungsverfahren nach Anspruch 8 zur Herstellung einer C-funktionalisierten und N-monofunktionalisierten Verbindung, eines Salzes, Solvats oder Gemischs davon, das folgende Schritte umfasst:
(i) Substitution eines Stickstoffatoms einer Verbindung der Formel (I) und/oder (II) durch eine Schutzgruppe R" gemäß der in der Formel (XIII) in Anspruch 10 angegebenen Definition, dann
(ii) Entschützung der Bisaminalbrücke mit Hydrazinhydrat oder Natriumhydroxid zum Erhalt der folgenden Verbindung der Formel (XIV), eines Salzes, Solvats oder Gemischs davon: 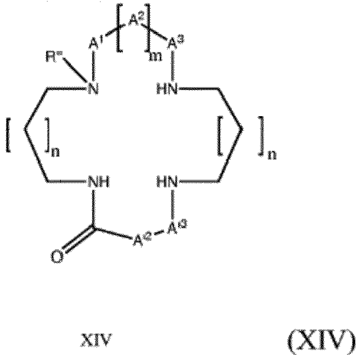 in der: A¹, A², A³, A'², A'³, m und n wie in Anspruch 1 oder 2 definiert sind, wobei die Gruppe R" wie in der Formel (XIII) in Anspruch 10 definiert ist,
(iii) gegebenenfalls Reduktion der Verbindung der Formel (XIV).

12. Herstellungsverfahren nach Anspruch 8 zur Herstellung einer C-funktionalisierten und N-monofunktionalisierten Verbindung, eines Salzes, Solvats oder Gemischs davon, das folgende Schritte umfasst:
(i) Reduktion einer cyclischen Verbindung der Formel (I) und/oder (II) gemäß einem der Ansprüche 1 bis 3 oder eines Gemisch davon mit einem selektiven Reduktionsmittel, dann
(ii) Substitution an zwei nicht benachbarten Stickstoffatomen des Tetrastickstoffrings der reduzierten Formel (I) oder (II) bzw. der reduzierten Formel (I) und (II) im Falle eines Gemischs oder der Formel (III) durch R"-Gruppen und
(iii) Entschützung der Bisaminalbrücke mit Hydrazinhydrat oder Natriumhydroxid zum Erhalt der folgenden Verbindung der Formel (XV), eines Salzes, Solvats oder Gemischs davon: 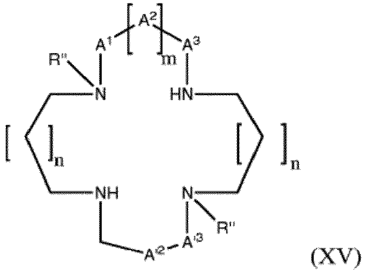 in der: A¹, A², A³, A'², A'³, m und n wie in Anspruch 1 oder 2 definiert sind, wobei die Gruppen R", die gleich oder verschieden sind, wie in der Formel (XIII) in Anspruch 11 definiert sind,
(iv) und gegebenenfalls dann, wenn R" eine Schutzgruppe für die Aminfunktion ist:
- Substitution von zwei unsubstituierten sekundären Aminen der Verbindung der Formel (XV) durch Gruppen R"', die gleich oder verschieden sind und für eine funktionelle Gruppe R" oder einen Vorläufer einer funktionellen Gruppe R" gemäß der in Anspruch 11 angegebenen Definition stehen, dann
- Entschützung der R"-Schutzgruppen.

13. Herstellungsverfahren nach Anspruch 8 zur Herstellung einer Verbindung der folgenden Formel (XVI), eines Salzes, Solvats oder Gemischs davon: 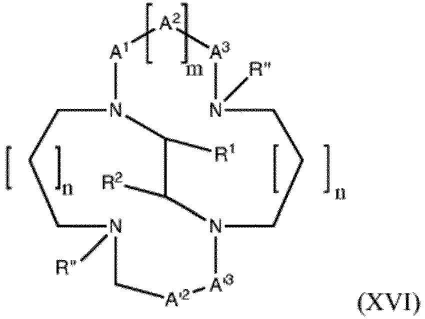 in der: A¹, A², A³, A'², A'³, R¹ und R², m und n wie in Anspruch 1 oder 2 definiert sind, wobei die Gruppen R", die gleich oder verschieden sind, wie in Formel (XIII) in Anspruch 11 definiert sind, wobei das Verfahren folgende Schritte umfasst:
(i) Reduktion der Ketonfunktion einer Verbindung der Formel (I) und/oder (II) gemäß einem der Ansprüche 1 bis 3 mit einem selektiven Reduktionsmittel, dann
(ii) Substitution der sekundären Amine der reduzierten Verbindung der Formel (I) und/oder (II) und schließlich
(iii) teilweise Reduktion der Bisaminalbrücke mit einem selektiven Reduktionsmittel.

14. Herstellungsverfahren nach Anspruch 8 zur Herstellung einer C-funktionalisierten und N-mono-und gegebenenfalls -difunktionalisierten Verbindung, eines Salzes, Solvats oder Gemischs davon, das folgende Schritte umfasst:
(i) Substitution eines sekundären Amins der Verbindung der Formel (I) und/oder (II) durch eine Gruppe R" gemäß der in Formel (XIII) in Anspruch 11 angegebenen Definition, dann
(ii) teilweise Reduktion der Bisaminalbrücke mit einem selektiven Reduktionsmittel zum Erhalt einer Verbindung der Formel (XVII), eines Salzes, Solvats oder Gemischs davon: 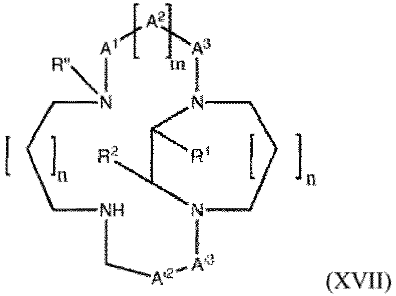 in der: A¹, A², A³, A'², A'³, R¹ und R², m und n wie in Anspruch 1 oder 2 definiert sind, wobei die Gruppe R" wie in Formel (XIII) in Anspruch 11 definiert ist,
und gegebenenfalls: (iii) Substitution des sekundären Amins der Verbindung der Formel (XVII) durch eine Gruppe R" gemäß der in Formel (XIII) in Anspruch 11 angegebenen Definition zum Erhalt einer Verbindung der Formel (XVII'), eines Salzes,
Solvats oder Gemischs davon: 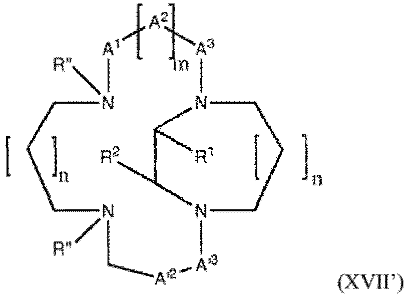 in der: A¹, A², A³, A'², A'³, R¹ und R², m und n wie in der in einem der Ansprüche 1 bis 3 definierten Formel (I) oder (II) definiert sind, wobei die Gruppen R", die gleich oder verschieden sind, wie in Formel (XIII) in Anspruch 11 definiert sind.

## Claims

1. Compound meeting the formula (I) or (II), its salts or solvates: 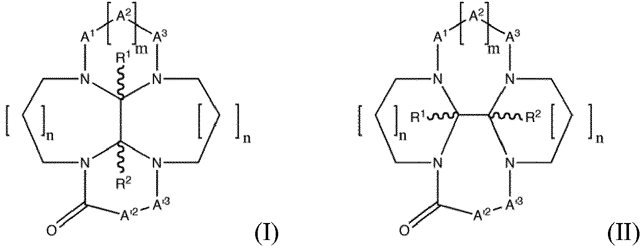 formulae (I) and (II), wherein:
R¹ and R², identical or different, represent a hydrogen atom, a C1-C4 alkyl group, preferably methyl, or, together with the carbon atoms of the bisaminal bridge to which they are linked, they can form a saturated or unsaturated, six-membered hydrocarbon cyclic group,
m and n, identical or different, are equal to 0 or to 1;
A¹, A², A³, A'², A'³, identical or different, are chosen from the groups CH₂, CHR and C=O; on condition that A'² or A'³ designates a CHR group;
the group or groups R, identical or different, ionic or non-ionic functional group are chosen from amongst:
- a hydrocarbon group, saturated or unsaturated, straight or branched, cyclic or acyclic, comprising 1 to 30 carbon atoms, preferably linear and 8 to 20 carbon atoms, and comprising, as the case may be, one or more heteroatoms,
- a group V, V being chosen from amongst a halogen, a nitrile, NR⁵R⁶, NO₂, OR⁷, COX, NHCOX, -CONR⁵R⁶, (O)ₛPO(OR⁸)(OR⁹), s being an integer ranging from 0 to 1, X being a halogen or a group OR⁵; R⁵, R⁶, R⁷, R⁸ and R⁹ being chosen from amongst a hydrogen atom and a straight or branched, saturated or unsaturated C₁-C₃₀ alkyl group,
- a group W, W being a cyclic hydrocarbon group that can be monocyclic, bicyclic or tricyclic, saturated or unsaturated, the cycle or cycles comprising 5 to 6 carbon atoms, and at least one of them being capable of being substituted by one or more groups chosen from amongst N-chlorosuccinimide, P(C₆H₅)₂, P(O)(C₆H₅)₂, (CH₂)ₚ V, p being an integer ranging from 0 to 4 and/or being capable of forming, with one or more carbon atoms of the cycle, one or more ketone functions,
- a group Y, Y being a heterocyclic group, capable of being monocyclic, bicyclic or tricyclic, saturated or unsaturated, the cycle or cycles comprising five to six members, and at least one of them being capable of being substituted on at least one or more carbon atoms or heteroatoms by one or more groups chosen from amongst the groups V and W, a straight or branched, saturated or unsaturated, C₁-C₃₀, alkyl group substituted by one or more groups V and/or W, a trityl group; the heterocycle group being capable of forming, with one or more carbon atoms of the cycle, one or more ketone functions,
- a straight or branched, saturated or unsaturated C₁-C₃₀ alkyl group substituted by one or more groups, V, W, and/or Y
- hydrophobic groups chosen form amongst saturated or unsaturated, straight or branched, cyclic or acylic hydrocarbon groups comprising at least one linear hydrocarbon chain having 8 to 30 carbon atoms,
- hydrophilic groups comprising at least one glycol ethylene group,
- lipidic or phospholipidic groups,
- fluorescent groups,
- ionic groups chosen from amongst organic groups of the guadinidium or arsenium type,
- groups bearing at least one primary, secondary or tertiary amine function, possibly protected,
- groups bearing at least one alcohol function,
- groups bearing at least one alkene and/or alkyne function,
- groups bearing at least one amide function,
- groups bearing at least cyano, nitrile, CF₃ function,
- groups bearing at least one acid, ester, aldehyde function.

2. Compound according to claim 1, **characterized in that** A'² designates a CHR group, R being as defined in claim.

3. Compound according to any one of the above claims, **characterized in that** it meets the following formula (III), one of its salts or solvates: 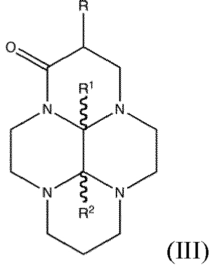 wherein R¹ and R² are as defined in the claim 1; R is a group
(CH₂)ᵢX with i as an integer equal to zero or one, X being a group chosen from amongs a halogen atom, a hydroxyl, CH₂OH, COOH, COOCH₃, COOC₂H₅.

4. Method for preparing a compound as defined in any one of the claims 1 to 3, **characterized in that** it comprises:
(i) a step during which a tetramine having formula (VII) is made to react 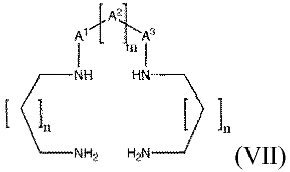 wherein: A¹, A², A³ are chosen from amongst from the groups CH₂, CHR and C=O, m and n, identical or different, are equal to 0 or 1,
with a dicarbonyl compound having formula (IV): 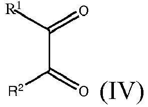 wherein: R¹ and R², identical or different, represent a hydrogen atom, a C₁-C₄ alkyl group, preferably methyl, or can together form, with the carbon atoms to which they are linked, a saturated or unsaturated cyclic hydrocarbon group with six carbon atoms, such as a phenyl or cyclohexane group, and preferably a cyclohexane group
to form a bisaminal intermediate having formula (VIII) or (IX) or a mixture of these compounds: 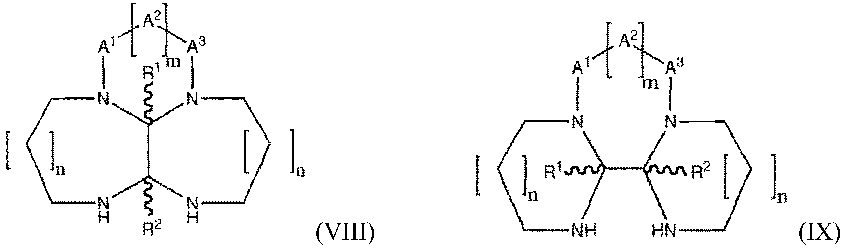 formulae (VIII) and (IX) wherein: A¹, A², A³, m, n are as defined in the formula (VII) and R¹ and R² are as defined in the formula (IV)
(ii) then a step during which the intermediate or intermediates obtained are made to react with a cyclizing agent meeting the formula (VI): 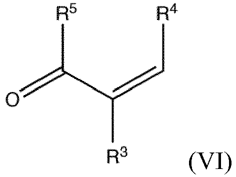 wherein:
- R³ and R⁴, which are identical or different, are a group R as defined in the formula (I) or (II) defined in any one of the claims 1 to 4;
- R⁵ represents a hydroxyl, a halogen, an alcoxyl; or
- R⁵ can form a cyclic group with R³ or R⁴.

5. Method for preparing a compound as defined in any one of the claims 1 to 3, **characterized in that** it comprises :
(i) a step during which at least two equivalents of ethylene diamine or propylene diamine are made to react with at least one equivalent of a dicarbonyl compound having formula (IV) as defined in the preceding claim,
to form a bisaminal intermediate having formula (V) or (V') or a mixture of these compounds: 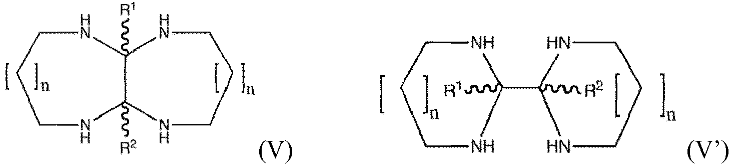 formulae (V) and (V') wherein R¹ and R² are as defined in the formula (IV) defined in the preceding claim;
(ii) then a step during which the intermediate obtained is made to interact with a first cyclizing agent having the formula (VI) as defined in the preceding claims,
(iii) and a step during which the novel intermediate obtained is made to react with the second cyclizing agent having the formula (VI) as defined in the preceding claims, or being a Q-CH₂CH₂CH₂-Q' compound with Q and Q' chosen amongst leaving groups such as halogens or a tosylate group
where the steps (ii) and (iii) can be performed simultaneously or successively.

6. Method according to claim 4 or 5, **characterized in that** the cyclizing agent having the formula (VI) is such that R⁵ forms a cyclic group with R³ or R⁴ meeting one of the formulae (X) or (XI): 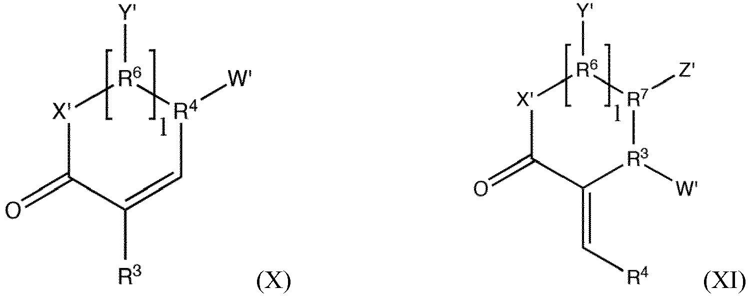
formula (X) wherein: R³ is a group R as defined in the formula (I) or (II) defined in any one of the claims 1 to 3.
X' is an oxygen atom,
Y' and W', identical or different, are chosen from amongst a hydrogen atom, a straight or branched, saturated or unsaturated C₁-C₃₀ alkyl group,
1 is equal to 0 or 1, with
- when 1 is equal to 0, the group X' is linked to the group R⁴ and R⁴ forming with W' a group CH₂ or C=O,
- when 1 is equal 1, R⁴ and R⁶ together form, with the groups Y' and W', the groups CH₂-CH₂, CH=CH, CH₂(C=O), (C=O)CH₂ or a phenyl group;
formula (XI) in which: R⁴ is a group R as defined in the formula (I) or (II) defined in any one of the claims 1 to 3,
X' is an oxygen atom,
W', Y' and Z', identical or different, are chosen from amongst a hydrogen atom, a straight or branched, saturated or unsaturated C₁-C₃₀ alkyl group.
R³, R⁶ and R⁷ designate CH₂ groups,
1 is equal to 0 or 1, with
- when 1 is equal to one,
R³ and R⁷ can together form, with the groups Z' and W', the groups: CH₂-CH₂, CH=CH, or a phenyl group, and/or
R⁶ and R⁷ can together form, with the groups Y' and Z', the groups: CH₂-CH₂, CH=CH, or a phenyl group
- when 1 is equal to zero, the group X' is linked to the group R⁷.

7. Method according to any one of the claims 4 to 6 , **characterized in that** the steps (i), (ii) and/or (iii) take place in a protic medium and/or are done without intermediate separation or purification steps.

8. Method for preparing a C-functionalized and/or N-functionalized tetranitrogen cyclic compound, derived from cyclam, from a compound having formula (I), (II), one of its salts, solvates or their mixture, as defined in any one of the claims 1 to 3, comprising at least one of the following steps;
A) substituting one or more nitrogen atoms of the compound (I) and/or (II) by one or more groups R", identical or different, preferably identical, where R" can designate any functional group, or any precursor of a functional group R", protected or not, as defined in one of the claims 1 to 3, or any amine function protector group,
B) reducing one or more ketone functions in alpha position of one of the four nitrogen atoms of the compound (I) and/or (II), said compound(s) (I) and/or (II) being possibly modified by one or more steps A) and/or C).
C) deprotecting the bisaminal bridge of the compound(s) (I) and/or (II), said compound(s) (I) and/or (II) being possibly modified by one or more steps A) and/or B), the steps B) and C possibly being done simultaneously or separately,
said C-functionalized and/or N-functionalized tetranitrogen cyclic compound, derived from cyclam having a formula chose amongst 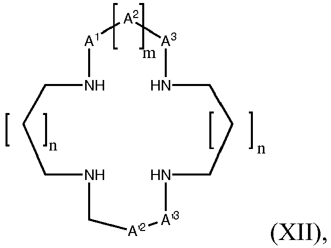 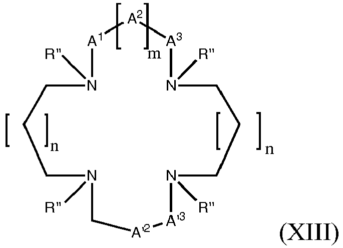 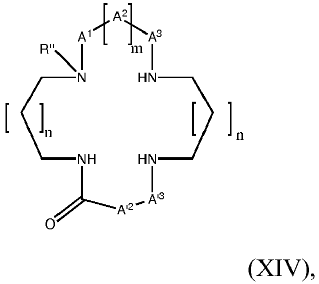 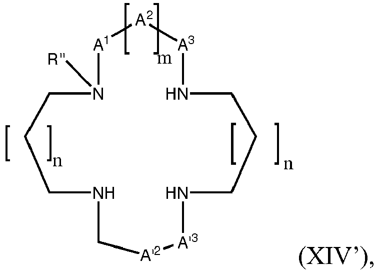 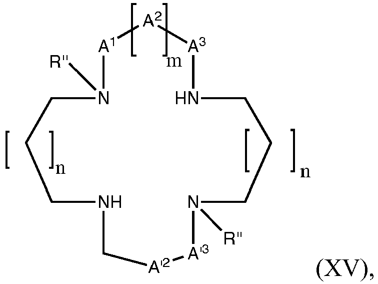 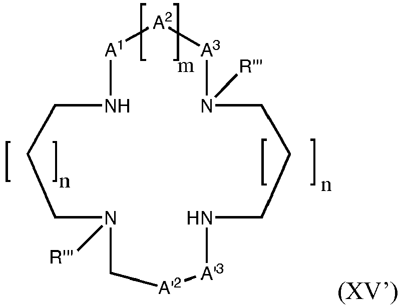 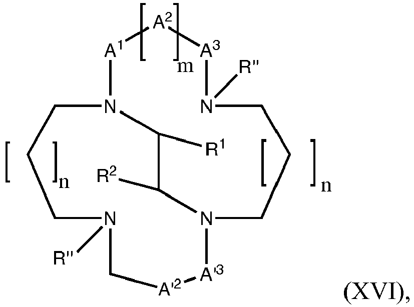 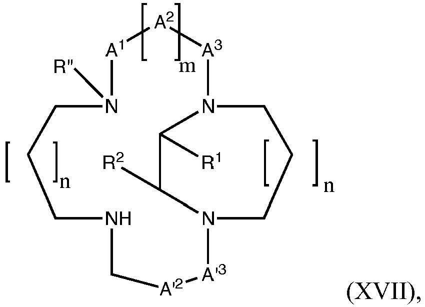 and 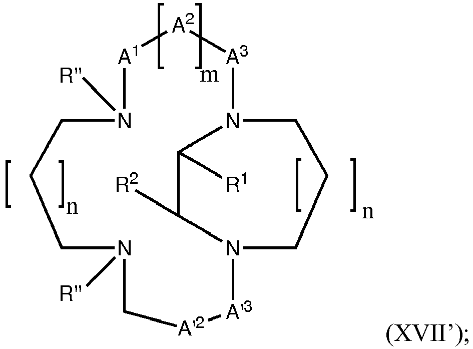 wherein: A¹, A², A³, A'², A'³, R¹ et R², m and n, are as defined in claim 1 or 2 groups R", which are identical or different, designate a functional group R or any R of a functional group, protected or not, as defined in one of the claims 1 to 2, or a protector group of a nitrogen atom of the tetranitrogen cyclic compound,
groups R"', identical or different, preferably identical designating a functional group R" or any precursor of a functional group R", protected or not.

9. Method for preparing according to claim 8 of a compound of formula (XII), one of its salts, solvates or mixture: 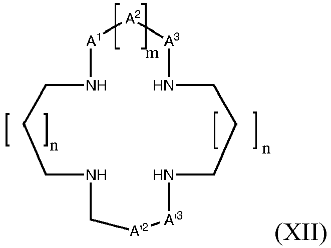 wherein: A¹, A², A³, A'², A'³, m and n, are as defined in claim 1 or 3, said method comprising a step for reducing the compound having formula (I) and/or (II) as defined according to any one of the claims 1 to 2.

10. Method for preparing, according to claim 8 a compound having the following formula (XIII), one of its salts, solvates or mixture: 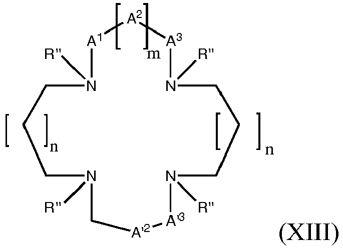 wherein: A¹, A², A³, A'², A'³, m and n are as defined in claim 1 or 2 where the groups R", which are identical or different, designate a functional group R or any precursor R of a functional group, protected or not, as defined in one of the claims 1 to 3, or a protector group of a nitrogen atom of the tetranitrogen cyclic compound, said method comprising the following steps:
i) reducing a compound having formula (I) or (II) as defined here above or their mixture, by means of at least one reducing agent, and finally
ii) substituting, simultaneously or successively, on the four nitrogen atoms of the tetranitrogen cycle of the compound having a reduced formula (I) and/or (II) by groups R", that are identical or different.

11. Method for preparing, according to claim 8, a C-functionalized coumpound and N-mono-functionalized compound, one of its salts, solvates or their mixture, comprising the following steps:
(i) substituting a nitrogen atom of a compound having formula (I) and/or (II) by a protective group R" as defined in the formula (XIII) in claim 10, then
(ii) deprotecting the bisaminal bridge with hydrazine hydrate or sodium hydroxide, to obtain the following compound having formula (XIV), one of its salts, solvates or mixture: 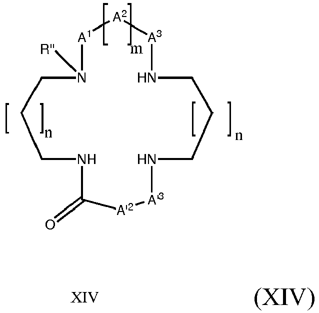 wherein: A¹, A², A³, A'², A'³, m and n are as defined in claim 1 or 2, the group R" is as defined in the formula (XIII) in claim 10,
(iii) possibly reducing the compound having formula (XIV).

12. Method for preparing, according to claim 8, a C-functionalized and N-mono-functionalized compound, one of its salts, solvates or mixture, comprising the following steps:
(i) reducing a cyclic compound having formula (I) and/or (II), as defined in one of the claims 1 to 3, or their mixture, by means of a selective reducing agent, then
(ii) substituting on two non-adjacent nitrogen atoms of the tetranitrogen cycle having formula (I) or (II) or (I) and (II) reduced in case of mixture, or (III) by groups R", and
(iii) deprotecting the bisaminal bridge with hydrazine hydrate or sodium hydroxide to obtain a compound having the following formula (XV), one of its salts, solvates or mixture: 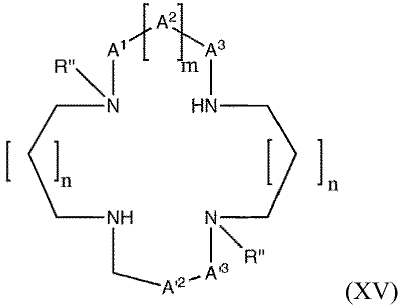 wherein: A¹, A², A³, A'², A'³, m and n are as defined in claim 1 or 3; the groups R", which are identical or different, are as defined in the formula (XIII) in claim 11,
(iv) and possibly when R" is an amine function protector group:
- substituting the two non-substituted secondary amines of the compound (XV) by groups R"', identical or different, designating a functional group R" or any precursor of a functional group R", protected or not, as defined in claim 11, the, protected or not, as defined in claim 11, then
- deprotectiing the protector groups R".

13. Method for preparing, according to claim 8, a compound having the following formula (XVI), one of its salts, solvates or mixture: 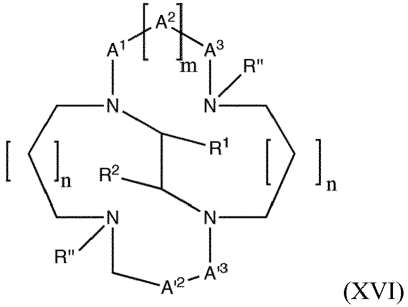 wherein: A¹, A², A³, A'², A'³, R¹ and R², m and n are as defined in the formula (I) or (II), the groups R", which are identical or different are as defined in claim 11,said method comprising the following steps
(i) reducing the ketone function of a compound having formula (I) and/or (II) as defined in one of the claims 1 to 3, by means of a selective reducing agent, then
(ii) substituting the secondary amines of the compound having the reduced formula (I) and/or (II), and finally
(iii) partially reducting the bisaminal bridge by means of a selective reducing agent.

14. Method for preparing a C-functionalized and and N-(mono and possibly di)-functionalized compound, one of its salts, solvates or mixture, comprising the following steps:
(i) substituting a secondary amine of the compound having formula (I) and/or (II) by a group R" as defined in the formula (XIII) in claim 11, then
(ii) partially reducing the bisaminal bridge by means of a selective reducing agent, to obtain a compound having the formula (XVII), one of its salts, solvates or mixture: 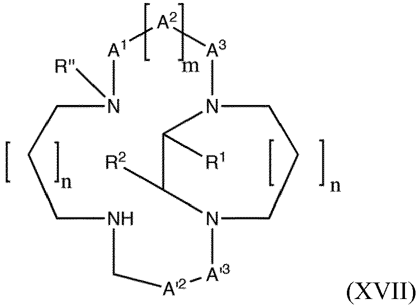 wherein: A¹, A², A³, A'², A'³, R¹ and R², m and n are as defined in the formula (I) or (II), the group R" is as defined in the formula (XIII) in claim 11,
and possibly (iii) substituting the secondary amine of the compound (XVII) by a group R" as defined in the formula (XIII) defined in the claim 11, to obtain a compound having the formula (XVII'), one of its salts, solvates of mixture: 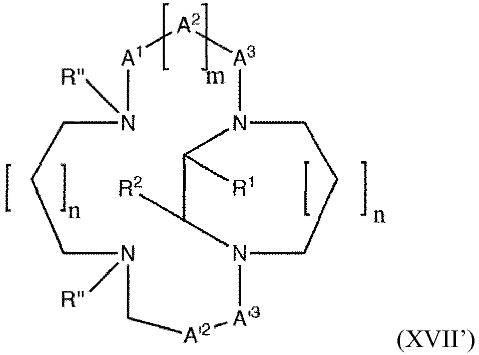 wherein: A¹, A², A³, A'², A'³, R¹ and R², m and n are as defined in the formula (I) or (II) defined in any one of the claims 1 to 3ten, the groups R", which are identical or different, are as defined in claim 11. 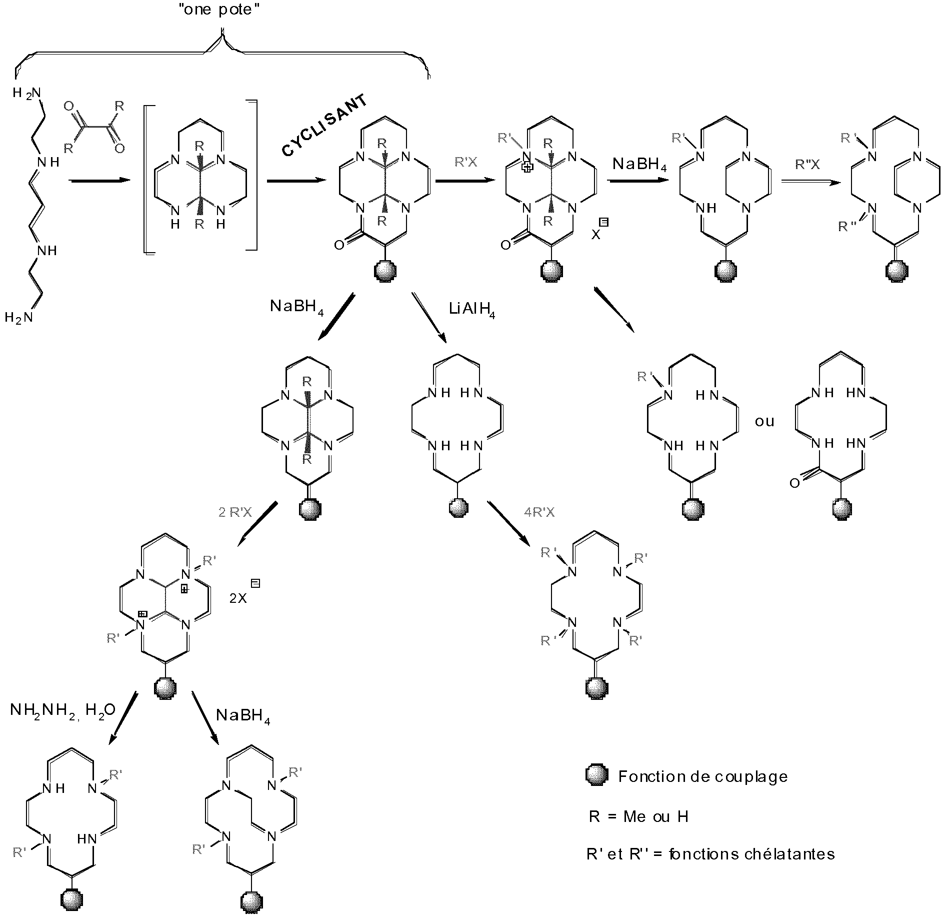 "one pot operation"
Cyclizing
Fonction de couplage = Coupling function
R' and R" = chelating functions
